# EUROPÄISCHE PATENTSCHRIFT

(11) **EP 3 281 615 B1**
(45) Veröffentlichungstag und Bekanntmachung des Hinweises auf die Patenterteilung: **24.11.2021**
(21) Anmeldenummer: 17185363.3
(22) Anmeldetag: 08.08.2017
(51) Int. Cl.: A61F 13/00, A61M 27/00, A61M 1/00

(54) **MEDIZINISCHES KIT ZUR VERWENDUNG BEI DER UNTERDRUCKTHERAPIE VON ENDOLUMINALEN WUNDSTELLEN IM BEREICH DES GASTROINTESTINALTRAKTES**
MEDICAL KIT FOR USE IN NEGATIVE PRESSURE THERAPY OF ENDOLUMINAL WOUND SITES IN THE GASTROINTESTINAL TRACT
TROUSSE MÉDICALE DESTINÉE À UTILISER LORS DU TRAITEMENT PAR PRESSION NÉGATIVE DES PLAIES ENDOLUMINALES DANS LA RÉGION DU TRACTUS GASTRO-INTESTINAL

(30) Priorität: 10.08.2016 DE 102016114817
(43) Veröffentlichungstag der Anmeldung: 14.02.2018
(73) Patentinhaber: Paul Hartmann AG, 89522 Heidenheim (DE)
(72) Erfinder: Görl Dr., Rudolf, 67853 Obernburg (DE); Eckstein Dr., Axel, 89522 Heidenheim (DE); Schulz, Heike, 73037 Göppingen (DE)

(56) Entgegenhaltungen:
- EP-A1- 1 572 286
- DE-A1-102008 061 536
- DE-A1-102013 202 849

## Beschreibung

Die Erfindung betrifft ein medizinisches Kit zur Verwendung bei der Unterdrucktherapie von endoluminalen Wundstellen im Gastrointestinaltrakt.

Unterdrucktherapie von Wunden, im Fachbereich auch bezeichnet als topische negative Druckbehandlung (TNP = topical negative pressure) oder NPWT (negative pressure wound therapy), ist seit langem bekannt, findet aber insbesondere seit Mitte/Ende der 1990er Jahre in zunehmendem Maße Anwendung. Dabei wird üblicherweise ein Wundfüllmaterial in die Wunde eingelegt, das Wundgebiet mit einer Folie abgedeckt und mittels eines Drainageschlauchs und einer Vakuumpumpe ein Unterdruck im Wundraum erzeugt. Durch die Verwendung des Wundfüllmaterials wird der Druck gleichmäßig über die Wunde verteilt. Der Unterdruck bewirkt, insbesondere in der initialen Phase der Wundbehandlung, eine effektive Wundreinigung durch Abtransport von Wundexsudat. Als weitere Vorteile werden, insbesondere in der nachfolgenden Granulationsphase, Förderung der Bildung von Granulationsgewebe und Verminderung der Wundödembildung genannt.

Unterdrucktherapie kann bei akuten oder bei chronischen Wunden eingesetzt werden. Hierbei können auch infizierte Wunden erfolgreich behandelt werden. Insbesondere wird die Unterdrucktherapie eingesetzt bei Geschwüren (Ulcus, z.B. Druckgeschwüren, diabetischen Geschwüren, neuropathischen Geschwüren oder venös bedingten Geschwüren), Druckwunden (Dekubitus), traumatischen Wunden, therapieresistenten Wunden, postoperativen Wunden, sondierten Fisteln sowie Hautlappen und Hauttransplantaten. Chronische Wunden sind durch eine verlangsamte oder fehlende Wundheilung gekennzeichnet.

Vorrichtungen zur Unterdrucktherapie von Wunden sind entsprechend im Stand der Technik bekannt. Geeignete Unterdrucktherapiegeräte und Zubehör werden beispielsweise in den Dokumenten WO2011018132, WO2011018133, WO2012156140, WO2012156174, WO2012163617 und WO2013159904 beschrieben. In den Wundraum können beispielsweise die aus den Dokumenten WO2010072309, WO2012022484, WO2011072840, WO2012167942 oder WO2011003521 bekannten Wundauflagen eingebracht werden. Als besonders vorteilhaft in der Unterdruck-Wundbehandlung hat sich nach der Erfahrung des Anmelders eine Wundauflage aus einem offenzelligen Polyester-Polyurethanschaum, die in der EP2515812B1 (aus WO2012022485) näher beschrieben ist, erwiesen.

Geräte zur Unterdrucktherapie von Wunden sind auch kommerziell erhältlich und umfassen kleine, tragbare Geräte, die den Patienten eine gewisse Mobilität ermöglichen bis hin zu stationär zu verwendenden Geräten, etwa in Langzeitpflegeeinrichtungen. Auch für die Unterdrucktherapie geeignetes Zubehör wie beispielsweise Wundauflagen, weitere Verbandkomponenten oder Anschlussmittel sind kommerziell erhältlich.

Ein neues Anwendungsgebiet eröffnet sich für die Unterdrucktherapie bei der Behandlung von Wundstellen im Lumen des Verdauungstraktes. Indikationen für eine Unterdruckapplikation im Gastrointestinaltrakt umfassen Entzündungen, Verätzungen, Anastomose-Insuffizienzstellen und Tumorexzissionsstellen. Chirurgische Anastomosen entstehen nach Resektion von Abschnitten des Verdauungstrakts. Eine gastrointestinale Anastomoseinsuffizienz, also eine mangelnde Dichtheit der chirurgisch geschaffenen Verbindung zwischen Abschnitten des Verdauungstraktes, kann als postoperative Komplikation beispielsweise auf eine entzündungs- oder ischämisch bedingte Nahtinsuffizienz zurückgehen. Bei einem Austritt des Inhalts von Magen oder Darm in eine Körperhöhle drohen lebensgefährliche Entzündungen, beispielsweise eine lebensgefährliche Bauchfellentzündung (Peritonitis) bei einem Austritt von Darminhalt in die Bauchhöhle. Weitere mögliche Komplikationen umfassen Abszesse und schlimmstenfalls eine Sepsis. Zu den bisherigen Behandlungsansätzen beim Auftreten von Anastomoseinsuffizienzen gehören beispielsweise eine weitere chirurgische Intervention, die Platzierung eines Stents oder eine medikamentöse Behandlung. Die Erfolgsaussichten der genannten Therapien sind unbefriedigend.

Deutlich verbesserte Heilungschancen bietet inzwischen eine Behandlungsoption zur Versorgung von Anastomoseinsuffizienzen des Gastrointestinaltraktes, welche auf einer endoluminal gesetzten Schaumdrainage bei gleichzeitiger Applikation von Unterdruck beruht. Diese Behandlungsmethode wird auch als endoluminale Vakuumtherapie (EVT) oder als endoluminale Unterdrucktherapie bezeichnet. Eine Übersicht zu neuartigen Behandlungsverfahren bei Perforationen des oberen Gastrointestinaltraktes findet sich beispielsweise in Menningen R, Senninger N und Laukötter MG (2014) World Journal of Gastroenterology, Vol. 20, No. 24, S. 7767 - 76. Fallberichte zum erfolgreichen Einsatz der endoluminalen Vakuumtherapie bei ösophagaler Perforation werden beispielsweise in Hampe J (2014) Annals of Thoracic Surgery, Vol. 97, No. 3, S. 1029 - 1035 beschrieben.

Anastomoseinsuffizienzen des Gastrointestinaltraktes umfassen kleinere Undichtigkeiten an der Nahtstelle bis hin zu einer ausgeprägten Abzesshöhle. Im Falle von Kavitäten, welche seitlich aus dem Lumen der Verdauungsorgane abzweigen können, wurden Volumina von 40 cm³ bis hin zu 160 cm³ beschrieben, wobei sich die Aussackung auf eine Länge von bis zu 8 cm erstrecken kann. Die Öffnungen der Kavität weisen typischerweise Durchmesser im Bereich von 2 - 4 cm auf.

Bei einer beabsichtigten Behandlung einer endoluminalen Wundstelle mittels Unterdruck muss ein mit dem stromaufwärts gelegenen Ende eines Absaugschlauches verbundener Fluidsammelkörper geeigneter Größe bereitgestellt und passgenau zur Zielstelle gebracht werden.

Die Ausdrücke "stromaufwärts" und "stromabwärts" sind im Zusammenhang mit der vorliegenden Anmeldung auf den Fluidstrom bezogen, welcher bei der hier beschriebenen Unterdrucktherapie endoluminaler Wundstellen ausgehend von der Wundstelle bzw. Wundkavität aus dem Körper heraus und weiter zur Unterdruckpumpe hin bewegt wird. Das stromaufwärts gelegene Ende des Absaugschlauches befindet sich demnach während der Unterdrucktherapie an der Wundstelle oder nahe der Wundstelle. Das stromabwärts gelegene Ende des Absaugschlauches ragt bei der Anwendung der Vorrichtung normalerweise aus dem Körper des Patienten heraus.

Ein mit einem Absaugschlauch verbundener Fluidsammelkörper wird im Folgenden auch als "Saugkörper" bezeichnet.

Als Fluidsammelkörper wird üblicherweise ein Schwammkörper, welcher insbesondere einen porösen Polymerschaumstoff umfasst, verwendet. Bei der Zielstelle kann es sich im einfacheren Falle um das Innere des Hauptlumens eines Abschnittes des Gastrointestinaltraktes handeln, beispielsweise um die Speiseröhre, um den Dickdarm oder um den Dünndarm.

Eine besondere Herausforderung für den behandelnden Arzt ergibt sich bei der Applikation des Saugkörpers in eine aus dem Hauptlumen abzweigende Aussackung. Eine derartige Aussackung kann insbesondere infolge einer Anastomoseinsuffizienz entstehen. Der Saugkörper kann nur dann in die Kavität eingeführt werden, wenn die Aussackung spiegelbar, d.h. mit einem Endoskop zugänglich ist. Die Größe des Fluidsammelkörpers muss an die Abmessung der Kavität angepasst sein. Die Abmessung der sackartig beschaffenen Kavität wird vor der Applikation des Saugkörpers endoskopisch ermittelt. Nach dem Einführen des Saugkörpers in das zu behandelnde, von der Speiseröhre oder von dem Darm abzweigende Lumen, wird das aus dem Patienten herausragende Ende des Absaugschlauches mit einer Unterdruckquelle verbunden und Unterdruck angelegt. Infolge des Unterdrucks kollabiert die zu behandelnde Kavität teilweise, wobei sich ein enger Kontakt zwischen der Wand der Kavität und dem Fluidsammelkörper einstellt. Der Kontakt zwischen Fluidsammelkörper und der Wand des Lumens stimuliert die Bildung von Granulationsgewebe. Wundfluide werden über den Fluidsammelkörper und den Absaugschlauch abgesaugt, so dass die Wunde permanent gesäubert wird und Infekte unter Kontrolle gehalten werden. Des Weiteren führt die Unterdruckapplikation zu einer kontinuierlichen Konstriktion der Ausstülpung. Der Saugkörper muss nach einigen Tagen kontinuierlicher Unterdruckapplikation immer wieder ersetzt werden, wobei die Größe des Fluidsammelkörpers bei fortschreitender Verkleinerung der Kavität schrittweise reduziert wird. Die Unterdruckbehandlung kann beendet werden, wenn die Aussackung weitestgehend zurückgegangen ist.

Im Falle einer Behandlung einer Anastomoseinsuffizienz ohne Aussackung wird die Therapie beendet, wenn die Leckage geschlossen ist.

Die Erfolgschancen des endoskopischen Eingriffs hängen wesentlich von der Auswahl eines passend dimensionierten Fluidsammelkörpers sowie von einer optimalen Platzierung des Saugkörpers in der Wundhöhle ab. Um den Saugkörper in die Kavität einzuführen, sind aus der medizinischen Praxis unterschiedliche Ansätze bekannt.

Die EP1572286-B1 beschreibt ein Behandlungssystem zur endoskopischen Wundversorgung, welches insbesondere zur Behandlung perianastomotischer Abszesse vorgesehen ist. Das Behandlungssystem umfasst einen Fluidsammelkörper, einen mit dem Fluidsammelkörper verbundenen Absaugschlauch sowie eine Einführhilfe (Inserter). Die Einführhilfe besteht aus einem dickeren Schlauch (Outer Sleeve bzw. Overtube) und aus einem dünneren Schlauch (Inner Sleeve bzw. Pusher). Der Overtube wird über ein bewegliches Endoskop in die Wundhöhle geschoben. Nach dem Entfernen des Endoskops wird der Fluidsammelkörper durch den Overtube geschoben und mithilfe des Pusher positioniert bzw. freigesetzt. Ein derartiges endoskopisches Behandlungssystem ist unter der Bezeichnung Endo-SPONGE® für den unteren Gastrointestinaltrakt und unter der Bezeichnung Eso-SPONGE® für den oberen Gastrointestinaltrakt im Handel erhältlich (Firma B. Braun Melsungen AG, Deutschland). Ein Einführsystem mit Positionierungshülse und Führungshülse ist beispielsweise auch aus der DE102009043472 bekannt.

Ein alternatives Verfahren zur präzisen Platzierung des Saugkörpers an der gewünschten Wundstelle beruht darauf, den am Saugkörper vorhandenen Schwamm mit einer am Ende eines endoskopischen Instrumentes angebrachten Zange zu ergreifen und dann mittels des Endoskops in die Wundtasche zu ziehen. Gemäß einer in der Praxis üblichen Vorgehensweise wird hierbei unmittelbar vor der endoskopischen Behandlung durch den behandelnden Arzt unter Einsatz chirurgischen Nahtmaterials am Wundschwamm eine Fadenschlaufe angebracht. Der Faden wird hierbei mit dem Schwamm und normalerweise auch mit dem Absaugschlauch vernäht, um ein Ausreißen des Schwamms zu vermeiden. Die EP2769744 beschreibt ein Medizinprodukt zur Drainage pathologischer Flüssigkeitsansammlungen, welches einen Fluidsammelkörper und einen mit dem Fluidsammelkörper verbundenen Absaugschlauch umfasst. Innerhalb des Absaugschlauches ist ein Faden vorhanden, welcher schlaufenförmig aus dem bei der Behandlung zur Wundhöhle hin zeigenden Ende des Absaugschlauches heraushängt. Die Fadenschlaufe dient als Fassschlaufe zur Positionierung des Saugkörpers mittels eines Endoskops.

Die DE102008061536A1 offenbart einen Kit zur Herstellung eines medizinischen Saugkörpers zur Entfernung von Wundflüssigkeiten aus menschlichen oder tierischen Körperhöhlen. Der Kit umfasst einen Saugkörper und eine Umhüllung für den Saugkörper.

Die endoskopische Einbringung des Saugkörpers in den Gastrointestinaltrakt erfolgt normalerweise über den Mund, so dass das Ende des Absaugschlauches zunächst aus dem Mund des Patienten herausragt. Üblicherweise wird der Schlauch dann vor der Beaufschlagung mit Unterdruck in die Nase transloziert, da dies für den Patienten weniger irritierend ist und der Schlauch im Nasenbereich zuverlässiger fixiert werden kann. Zur "Translokation" wird ein Hilfsschlauch über die Nase in den Rachenraum geführt. Der Hilfsschlauch kann dann mit dem Absaugschlauch verbunden werden. Hierzu werden üblicherweise die Schlauchenden von Absaugschlauch und Hilfsschlauch, welche einen unterschiedlichen Durchmesser aufweisen müssen, ineinander geschoben. Durch Ziehen an dem Hilfsschlauch wird der Absaugschlauch dann über die Nase herausgezogen. Bezüglich der Größenanpassung ist es derzeit in der Praxis üblich, einen kommerziell erhältlichen Schwammkörper vor der Anwendung mittels einer Schere oder mittels eines Skalpells an die endoskopisch ermittelten Abmessungen (Durchmesser und/oder Länge des Schwammkörpers) des zu behandelnden Wundtraumes anzupassen.

Der vorliegenden Erfindung lag die Aufgabe zugrunde, ein medizinisches Produkt bereit zu stellen, um die endoluminale Unterdrucktherapie weiter zu verbessern. Insbesondere sollte die Handhabung der endoskopischen Komponenten vereinfacht werden, die Dauer des endoskopischen Eingriffes verkürzt und die Anwendungssicherheit erhöht werden.

Die Erfindung löst die oben genannten Aufgaben durch die Bereitstellung eines medizinischen Kits zur Verwendung bei der Unterdrucktherapie von endoluminalen Wundstellen im Bereich des Gastrointestinaltraktes gemäß Anspruch 1.

Das erfindungsgemäße Kit umfasst mindestens zwei Saugkörper. Wie eingangs dargestellt, wird der Ausdruck "Saugkörper" im vorliegenden Zusammenhang allgemein für einen mit einem Absaugschlauch verbundenen Fluidsammelkörper verwendet, wobei es sich bei dem Fluidsammelkörper um einen Schwamm handelt, welcher insbesondere einen porösen Polymerschaum umfasst.

Jeder der mindestens zwei in dem Kit enthaltenen Saugkörper umfasst einen Schwammkörper. Dieser ist im Wesentlich zylinderförmig ausgebildet, wobei zusätzlich zum stromaufwärts gelegenen Ende hin und/oder zum stromabwärts gelegenen Ende hin abgeschrägt oder gebogen (kuppelförmig) verlaufende Abschnitte vorhanden sein können. Es können auch eine oder mehrere Abschnürungen vorhanden sein, insbesondere eine Abschnürung am stromaufwärts gelegenen Ende des Schwammkörpers und/oder eine Abschnürung am stromabwärts gelegenen Ende des Schwammkörpers. Bei dem Zylinder handelt es sich insbesondere um einen senkrechten Kreiszylinder. Der hier verwendete Begriff "im Wesentlichen zylinderförmig" umfasst auch solche senkrechten Kreiszylinder, welche eine oder zwei abgerundete oder abgeschrägte Kanten aufweisen. Jeder der Schwammkörper weist einen (gegenüber den anderen in dem Kit vorhandenen Schwammkörpern unterschiedlichen oder identischen) Durchmesser d und eine (gegenüber den anderen in dem Kit vorhandenen Schwammkörpern unterschiedliche oder identische) Länge I auf.

Die in den Kits enthaltenen Schwammkörper sind zum Auskleiden einer Wundhöhle und zur Aufnahme von Fluiden aus dieser Wundhöhle vorgesehen. Bei den aus der Wundhöhle aufzunehmenden Fluiden handelt es sich insbesondere um flüssige und zähflüssige Substanzen, beispielsweise um Blut, Schleim, Eiter, aber auch um von außen während der Behandlung zugeführte Flüssigkeiten. Der Schwammkörper umfasst vorzugsweise einen polymeren Schaumstoff, insbesondere einen offenzelligen Polymerschaum aus Polyurethan oder Polyvinylalkohol. Der Schwammkörper kann vorzugsweise auch vollständig aus einem polymeren Schaumstoff bestehen. Besonders bevorzugt wird ein Polymerschaumstoff aus einem Polyester-Polyurethan eingesetzt, wie beispielsweise in der EP2515812B1 beschrieben.

Weiterhin umfasst jeder der mindestens zwei in dem Kit enthaltenen Saugkörper einen mit dem Schwammkörper herstellerseitig unlösbar verbundenen Absaugschlauch.

Der Absaugschlauch dient zur Beaufschlagung der Wundstelle mit Unterdruck und zum Abtransport der von dem Fluidsammelkörper aufgenommenen Substanzen. Hierzu weist jeder der Absaugschläuche eine oder mehrere Öffnungen auf.

Vorzugsweise ist an dem bei Gebrauch des Saugkörpers aus dem Patienten herausragenden Ende des Absaugschlauchs ein Konnektor zum Verbinden des Absaugschlauches mit einer Unterdruckquelle vorhanden. Bei dem Konnektor handelt es sich insbesondere um einen Schnelltrennverbinder.

Erfindungsgemäß ist das Kit dadurch ausgezeichnet, dass sich jeder der in dem Kit vorhandenen Schwammkörper in mindestens einer der Größen ausgewählt aus Durchmesser d und/oder Länge I von jedem anderen der in dem Kit vorhandenen Schwammkörper unterscheidet. Die in dem Kit enthaltenen Bestandteile sollten vorzugsweise steril abgepackt vorliegen.

Mit dem erfindungsgemäß vorgeschlagenen Kit wird dem Anwender eine für die medizinische Praxis optimierte Auswahl von Saugkörpern für die endoluminale Unterdrucktherapie zur Verfügung gestellt, welche dem Anwender erlaubt, schnell und flexibel auf eine Wundsituation zu reagieren. Die Auswahl des hinsichtlich seiner Abmessungen geeigneten Saugkörpers aus dem Kit wird anhand einer vor dem Eingriff durchgeführten endoskopischen Vermessung der Wundstelle vorgenommen.

Der Kit umfasst mindestens zwei Schwammkörper, welche sich nur in einer einzigen Größe, ausgewählt aus Durchmesser d oder Länge I von jedem der anderen in dem Kit vorhandenen Schwammkörper unterscheiden. Bevorzugt unterscheiden sich alle in dem Kit vorhandenen Schwammkörper in nur einer einzigen Größe, ausgewählt aus Durchmesser d oder Länge I von jedem der anderen in dem Kit vorhandenen Schwammkörper. Erfindungsgemäß umfasst das Kit mindestens zwei Schwammkörper, welche eine identische Länge I und einen unterschiedlichen Durchmesser d aufweisen. Diese Ausführungsform erlaubt dem Arzt / der Ärztin insbesondere bei der Behandlung von Wundkavitäten im Bereich des Gastrointestinaltraktes eine besonders exakte Anpassung des Schwammkörpers an die Wundstelle.

Dies erweist sich nämlich deshalb als besonders praktisch, weil es für einen Anwender vergleichsweise aufwändiger und schwieriger ist, einen zylinderförmigen Schwammkörper in seinem Durchmesser d anzupassen. Dagegen ist es vergleichsweise einfacher, den Schwammkörper mittels einer Schere oder mittels eines Skalpells in seiner Länge I einzukürzen. Entsprechend wählt der Anwender nach der endoskopischen Vermessung der Wundstelle einen Saugkörper aus dem Kit aus, welcher hinsichtlich seines Durchmessers d optimal an die zu behandelnde Stelle angepasst ist. Anschließend nimmt der Anwender noch eine Längenanpassung durch Verkürzen des Schwammkörpers vor, falls dies erforderlich ist. Dabei wird erfinderseitig vorgeschlagen, dass das Kit insbesondere drei oder vier Saugkörper enthält. Die Länge jeder der Schwammkörper beträgt hierbei herstellerseitig (vor dem gegebenenfalls erforderlichen Zuschneiden durch einen Anwender) vorteilhaft 5 cm bis 15 cm, insbesondere 7 cm bis 11 cm.

Bei der hier genannten Ausführungsform unterscheiden sich die unterschiedlichen in einem Kit vorhandenen Schwammkörper also ausschließlich bezüglich ihres Durchmessers d. Dieser Durchmesser d liegt vorzugsweise im Bereich von 0,5 cm bis 10 cm. So könnten die Durchmesser d1, d2, d3 bei drei im Kit vorhandenen Schwammkörpern beispielsweise 2 cm (d1), 3 cm (d2) und 4,5 cm (d3) betragen. Besonders bevorzugt liegen die Durchmesser der insbesondere drei oder vier Schwammkörper im Bereich von 1 cm bis 5 cm.

Besonders bevorzugt unterscheiden sich die Durchmesser d der Schwammkörper (bei identischer Länge I) um einen festen Faktor f, welcher insbesondere zwischen 1,3 und 2,5 liegt. Als besonders vorteilhaft zur Versorgung einer Vielzahl von Wundhöhlen hat sich ein Faktor f zwischen 1,5 und 2 erwiesen.

So könnten beispielsweisen in einem Kit, welches vier Saugkörper enthält, Schwammkörper mit folgenden Durchmessern d1 bis d4 und mit der Länge I vorhanden sein:
d1 = 2 cm; d2 = 3 cm; d3 = 4,5 cm; d4 = 6,75 cm
I = 12 cm (herstellerseitig, also vor dem Zuschneiden durch einen Anwender)
Der Faktor f beträgt bei diesem Beispiel 1,5.

Gemäß einer weiteren alternativen Ausführungsform unterscheiden sich die Durchmesser d (d1, d2, bis dn) der Schwammkörper (bei identischer Länge I) um einen fixen Wert, beispielsweise jeweils um 2 cm.

So könnten beispielsweisen in einem Kit, welches fünf Saugkörper enthält, Schwammkörper mit folgenden Durchmessern d1 bis d5 und mit der Länge I vorhanden sein:
d1 = 1 cm; d2 = 2 cm; d3 = 3 cm; d4 = 4 cm; d5 = 5 cm
I = 10 cm (herstellerseitig, also vor dem Zuschneiden durch einen Anwender)
Der fixe Wert beträgt bei diesem Beispiel 1 cm.

Ein Kit, welches zwei oder drei der erfindungsgemäß vorgeschlagenen Saugkörper enthält, wobei sich die Schwammkörper ausschließlich in ihrem Durchmessern d1 und d2 (zwei Schwammkörper) bzw. d1, d2 und d3 (drei Schwammkörper) voneinander unterscheiden, ist besonders vorteilhaft. Das Kit kann zu vertretbaren Kosten hergestellt werden und deckt alle wesentlichen Wundsituationen ab. Jeder der zwei oder drei Schwammkörper weist dabei herstellerseitig (also vor dem Zuschneiden durch einen Anwender) eine Länge zwischen 5 und 15 cm auf.

Zur weiteren Verbesserung des Saugkörpers wird vorgeschlagen, am stromabwärts vorhandenen Ende des Absaugschlauches ein Konnektorelement anzubringen. Hierbei kann es sich um ein männliches oder um ein weibliches Konnektorelement handeln, welches zum Verbinden mit einem Gegenstück ausgebildet ist, nämlich zur Koppelung mit einem weiteren, an einem Hilfsschlauch vorhandenen Konnektorelement.

Entsprechend ist am Hilfsschlauch das komplementäre Konnektorelement angebracht: Der Hilfsschlauch weist ein weibliches Konnektorelement auf, wenn das Konnektorelement am Absaugschlauch männlich ist und umgekehrt. Die an Absaugschlauch und Hilfsschlauch angebrachten Konnektorelemente erleichtern bei einer geeigneten Ausgestaltung die schonende Translokation des Absaugschlauches vom Mund zur Nase des Patienten. Für die Ausgestaltung der Konnektorelemente ist es hierbei erforderlich, dass die maximalen Durchmesser der Konnektorelemente die Durchmesser der damit verbundenen Schläuche (Absaugschlauch und Hilfsschlauch) nicht wesentlich überschreiten. Die Konnektorelemente dürfen höchstens den 1,5-fachen, bevorzugt höchstens den 1,4-fachen Durchmesser, besonders bevorzugt höchstens den 1,2-fachen Durchmesser der Schläuche aufweisen (unter Durchmesser wird hierbei die maximale Ausdehnung senkrecht zur Achse der Schläuche verstanden). Gleichzeitig müssen die Übergänge zwischen Absaugschlauch und Konnektorelement ebenso wie die Übergänge zwischen Hilfsschlauch und Konnektorelement fließend ausgebildet sein, d.h. es darf keine Stufe oder Kante vorhanden sein. Vorgeschlagen wird somit ein Saugkörper zur Verwendung bei der Unterdrucktherapie von endoluminalen Wundstellen im Bereich des Gastrointestinaltraktes, umfassend einen Schwammkörper und einen mit dem Schwammkörper mittels Klebstoffauftrag unlösbar verbundenen Absaugschlauch, wobei der Absaugschlauch einen maximalen Durchmesser dₐ, aufweist und wobei an dem Absaugschlauch ein männliches oder ein weibliches Konnektorelement herstellerseitig angebracht ist, wobei das Konnektorelement einen maximalen Durchmesser d_{ka,} aufweist. Der Durchmesser dₖₐ darf höchstens das 1,5-fache des Durchmessers dₐ betragen und der Übergang zwischen Absaugschlauch und Konnektorelement muss fließend ausgebildet sein. Bevorzugt beträgt der Durchmesser dₖₐ höchstens das 1,4-fache, besonders bevorzugt höchstens das 1,2-fache des Durchmessers dₐ.

Ergänzend wird eine Gesamtheit aus Saugkörper (wie vorstehend näher bezeichnet) und einem Hilfsschlauch vorgeschlagen, wobei am Hilfsschlauch ein Konnektorelement vorzugsweise herstellerseitig angebracht ist, und wobei das am Hilfsschlauch vorhandene männliche oder weibliche Konnektorelement komplementär zu dem am Absaugschlauch vorhandenen Konnektorelement ausgebildet ist, so dass Hilfsschlauch und Absaugschlauch auf einfache Weise miteinander gekoppelt werden können. Der Durchmesser dₖₕ des am Hilfsschlauch vorhandenen Konnektorelementes darf gleichfalls höchstens das 1,5-fache, bevorzugt das 1,4-fache, besonders bevorzugt höchstens den 1,2-fache des Durchmessers dₐ betragen und der Übergang zwischen Hilfsschlauch und Konnektorelement muss fließend ausgebildet sein. Der maximale Durchmesser dₕ des Hilfsschlauchs darf gleichzeitig den Durchmesser dₐ des Absaugschlauches nicht übertreffen. Insbesondere sind die Durchmesser dₕ und dₐ gleich.

Hierbei bezeichnet die Variable dₐ den maximalen Durchmesser des Absaugschlauches, dₕ den maximalen Durchmesser des Hilfsschlauches, dₖₐ den maximalen Durchmesser des am Absaugschlauch angebrachten Konnektorelementes und dₖₕ den maximalen Durchmesser des am Hilfsschlauch angebrachten Konnektorelementes.

Gemäß einer bevorzugten Ausführungsform des erfindungsgemäßen medizinischen Kits ist bei jedem in dem Kit enthaltenen Saugkörper das vorstehend beschriebene Konnektorelement herstellerseitig unlösbar an dem Absaugschlauch befestigt. Für die praktische Anwendung ist es besonders vorteilhaft, wenn dem Kit zusätzlich ein oder mehrere Hilfsschläuche mit bereits herstellerseitig unlösbar befestigten Konnektorelementen beigefügt werden.

Nach einem weiterentwickelten Erfindungsgedanken wird ein Set vorgeschlagen, wobei das Set mindestens zwei Kits, wie vorliegend beschrieben, umfasst. Die von dem Set umfassten Kits unterscheiden sich bezüglich der Länge I der in den Kits enthaltenen Saugkörper voneinander. Unter einem Set wir hier eine Gesamtheit von mindestens zwei, bevorzugt zwei bis vier, erfindungsgemäßen Kits verstanden. Vorzugsweise umfasst das Set drei oder vier Kits. Das vollständige Set wird herstellerseitig bereitgehalten, so dass der Anwender bei Bedarf eines oder mehrere der in dem Set enthaltenen Kits bestellen kann. Um flexibel und schnell auf alle denkbaren Wundsituationen reagieren zu können, ist es noch vorteilhafter, wenn das vollständige Set nahe bei einem Anwender bereitgehalten wird, beispielsweise in dem Vorratsraum eines Krankenhauses oder insbesondere in einem Operationssaal.

Gemäß einer bei der Anwendung des Saugkörpers in der Unterdrucktherapie im Bereich des Gastrointestinaltraktes besonders bevorzugten Ausführungsform der Erfindung ist die Vorrichtung weiter dadurch gekennzeichnet, dass an der Vorrichtung eine bereits herstellerseitig angebrachte Fassschlaufe vorhanden ist. Die Fassschlaufe ist hierbei entweder aus einem Abschnitt des Schwammkörpers, oder aus einem Abschnitt des Absaugschlauchs oder aus einem mit dem Absaugschlauch unlösbar verbundenem Materialabschnitt geformt. Im Zusammenhang mit dieser Ausführungsform der Erfindung hat sich als besonders vorteilhaft erwiesen, wenn die Fassschlaufe aus einem aus dem Schwammkörper herausragenden Abschnitt des Absaugschlauch geformt wird, sofern der ein solcher Abschnitt des Absaugschlauchs vorhanden ist. Die Fassschlaufe dient zum Ergreifen der Vorrichtung und dann zum Führen des Schwammkörpers zur Wundstelle hin, wobei das Ergreifen und das zum Zielort hin translozieren üblicherweise durch ein endoskopisches Instrument unter Kamerakontrolle erfolgt. Hierzu weist das endoskopische Instrument an seinem Ende eine miniaturisierte Greifzange auf, welche die Fassschlaufe fest, jedoch reversibel packen und zum Zielort mitziehen kann. Es geht also bei der vorliegenden Ausführungsform insbesondere um eine bereits herstellerseitig an der Vorrichtung zur endoluminalen Unterdrucktherapie vorhandene Fassschlaufe, die mit einem medizinischen Instrument ergriffen werden kann. Unter dem Begriff "Fassschlaufe" wird im Zusammenhang mit der vorliegenden Erfindung allgemein eine griffförmige Struktur verstanden, welche geeignet ist, von einem an einem endoskopischen Instrument vorhandenen Greifmechanismus, insbesondere von einer Greifzange, reversibel festgehalten zu werden. Die Fassschlaufe soll hinsichtlich ihrer Abmessungen und ihrer Form an die an dem endoskopischen Instrument vorhandene Greifzange angepasst werden.

Im Gegensatz zu bisher üblichen Verfahren, bei denen der behandelnde Arzt einen Faden an den Saugkörper angebracht hat, ist die nach dieser bevorzugten Ausführungsform vorgestellte Methode schneller, zuverlässiger und sicherer. Der Arzt kann sich vollständig auf die bevorstehende Operation konzentrieren und muss sich nicht mit der Vorbereitung des Wundkörpers befassen. Die Fassschlaufe ist stabil und kann optimal auf das endoskopische Instrument abgestimmt werden. Ein unbeabsichtigtes Abfallen des Schwammes von dem endoskopischen Instrument wird weitestgehend vermieden. Die Fassschlaufe ermöglicht eine präzise Applikation des Schwammkörpers mittels eines flexiblen Endoskops. Ebenso gewährleistet eine auf das endoskopische Instrument abgestimmt Fassschlaufe eine unproblematische Freisetzung des Saugkörpers am Zielort: Im Gegensatz zu herkömmlichen Methoden wird die Gefahr reduziert, dass sich Fadenschlingen an der Greifzange des endoskopischen Instrumentes verheddern und so das Abkoppeln des Saugkörpers vom Instrument beeinträchtigen.

Gemäß einer ersten Variante der vorgenannten bevorzugten Ausführungsform wird die Fassschlaufe herstellerseitig aus einem Abschnitt des Absaugschlauchs geformt, welcher wundseitig aus dem Schwammkörper herausragt. Hierbei ist es besonders bevorzugt, dass die Fassschlaufe durch Stanzen einer Öffnung in den Absaugschlauch geformt wird. Die eingestanzte Öffnung kann in Längsrichtung des Absaugschlauches einen Durchmesser von 2 mm bis 200 mm aufweisen. Vorzugsweise beträgt ihre Länge zwischen 3 mm und 25 mm, wobei die Länge der Öffnung an die Abmessungen der Greifzange des endoskopischen Instrumentes abgestimmt ist. Der maximale Durchmesser der Stanzöffnung in Querrichtung hängt von der Dicke des Absaugschlauches ab. Der Durchmesser der eingebrachten Öffnung in Querrichtung darf den Durchmesser des Lumens des Absaugschlauches nicht überschreiten, um die Stabilität der Fassschlaufe zu gewährleisten. So darf beispielsweise bei einem Absaugschlauch mit einem äußeren Durchmesser von 5 mm und einem inneren Durchmesser des Lumens von 3 mm der Durchmesser der Stanzöffnung in Querrichtung 4 mm nicht überschreiten. Eine geeignete Abmessung für eine Fassschlaufe an einem medizinischen Absaugschlauch mit kreisförmigen Querschnitt und mit einem äußeren Durchmesser von 5 mm und einem Durchmesser des Lumens von 3 mm ist beispielsweise 5 mm in Längsrichtung und 4 mm in Querrichtung. Eine derartige Fassschlaufe lässt sich gut greifen und ist stabil. Das Einbringen der Stanzöffnung erfolgt nach üblichen Stanzverfahren und kann bei Raumtemperatur oder nach Erhitzen des Schlauches erfolgen.

Gemäß einer zweiten Variante derselben vorgenannten bevorzugten Ausführungsform wird die Fassschlaufe herstellerseitig aus einem mit dem Absaugschlauch unlösbar verbundenem Materialabschnitt geformt. Hierzu formt der Hersteller beispielsweise aus einem Kunststoffmaterial zunächst eine zum Greifen durch ein endoskopisches Instrument angepasste Fassschlaufe. Die Fassschlaufe kann beispielsweise aus einem Kunststoffmaterial geformt werden. Der eine Fassschlaufe umfassende Materialabschnitt wird dann unlösbar mit dem wundseitigen Ende des Absaugschlauchs verbunden. Eine unlösbare Verbindung kann beispielsweise durch Verpressen, durch Verkleben oder durch Vulkanisieren hergestellt werden. In der Praxis kann der Materialabschnitt neben der Fassschlaufe einen zapfenartigen Anteil umfassen, welcher zur unlösbaren Verbindung mit dem Absaugschlauch vorgesehen ist.

Nach einer weiteren vorteilhaften Ausgestaltung der zweiten Variante besteht der Materialabschnitt aus einem Faden, wobei der Faden beispielsweise aus einem textilen Material oder aus einem flexiblen Kunststoffmaterial hergestellt werden kann. Der Faden wird dann an der Außenseite des Absaugschlauches unlösbar befestigt, insbesondere durch Verkleben. Um eine besonders stabile Verbindung zwischen dem Absaugschlauch und dem Faden zu gewährleisten, sollte der Faden vorzugsweise aus einem Kunststoffmaterial bestehen.

Gemäß einer dritten Variante derselben vorgenannten bevorzugten Ausführungsform wird die Fassschlaufe herstellerseitig durch Stanzen einer Öffnung in den Schwammkörper, insbesondere durch Stanzen einer Öffnung in einen Polymerschaumstoff, geformt. Das Stanzen erfolgt vorzugsweise unter Hitzeeinwirkung. Um die Stabilität einer durch Stanzen aus dem Polymerschaumstoff geformten Fassschlaufe zu erhöhen, kann der Polymerschaumstoff nach dem Stanzen im Bereich der Fassschlaufe gehärtet werden. Eine Härtung des die Fassschlaufe umgebenden Materials ist beispielsweise durch Verpressen (insbesondere durch Verpressen unter Hitzeeinwirkung) oder durch chemische Behandlung mit einem aushärtbaren Polymer möglich. Zur Stabilisierung der durch Stanzen aus dem Schaum erzeugten Fassschlaufe können beispielsweise Polymergemische auf der Basis von Silikon, Polyurethan, Polyacetat, Polycarbonat, Polyethylen, Weich- Polyvinychlorid, Polyvinylalkohol oder thermoplastische Polymere verwendet werden. Die Modifikation des die Fassschlaufe umgebenden Materials sollte idealerweise zur Auflösung der Stege des Polymerschaumstoffs führen, so dass ein massives (nicht-porösen) Kunststoffmaterial gebildet wird, welches die Fassschlaufe umläuft. Dies hat den Vorteil, dass sich die Greifzange des endoskopischen Instrumentes beim Loslassen des Saugkörpers dann nicht mehr in den Stegen des Polymerschaumstoffs verfangen kann.

Bei den drei vorgenannten Varianten derselben bevorzugten Ausführungsform weist die Fassschlaufe vorzugsweise eine Länge zwischen 3 mm und 25 mm, besonders bevorzugt zwischen 3 mm und 15 mm, auf. Gemäß einer weiteren besonders bevorzugten Ausführungsform beträgt die Länge zwischen 10 mm und 25 mm. In der Praxis hat sich insbesondere eine Länge der Fassschlaufe von 12 mm (+/-2 mm) als besonders vorteilhaft erwiesen.

Gemäß einer weiteren Ausbildung der Erfindung, bei welcher eine zusätzliche Vereinfachung und weitere Zeitersparnis bei der Vorbereitung eines endoluminal Eingriffes erzielt werden kann, weist jeder der in dem Kit vorhandenen Absaugschläuche entlang seiner Achse mindestens einen ersten, einen zweiten und einen dritten Abschnitt auf. Herstellerseitig umschließt der Schwammkörper den ersten und den zweiten Abschnitt des Absaugschlauches. Der erste und der zweite Abschnitt des Absaugschlauchs verlaufen also innerhalb des Schwammkörpers. Der Absaugschlauch umfasst einen dritten Abschnitt, welcher nicht vom Schwammkörper umschlossen ist. Der dritte Abschnitt ist stromabwärts des ersten und des zweiten Abschnitts angeordnet. Der dritte Abschnitt des Absaugschlauchs wird aus dem Gastrointestinaltrakt des Patienten herausgeleitet.

Die zumindest eine im Absaugschlauch vorhandene Öffnung befindet sich bei dieser weiteren Ausbildung der Erfindung im Bereich des ersten Abschnittes des Absaugschlauchs. Weitere Öffnungen können im Bereich des zweiten und/oder des dritten Abschnittes des Absaugschlauchs vorhanden sein. Zusätzlich kann der Absaugschlauch eine an seinem wundseitigen Ende vorhandene endständige Öffnung aufweisen. Der Absaugschlauch fungiert somit als medizinischer Drainageschlauch. Bei der hier vorgeschlagenen weiteren Ausbildung der Erfindung ist es wesentlich, dass der Absaugschlauch im Bereich des ersten Abschnitts herstellerseitig mittels Klebstoffauftrag mit dem Schwammkörper unlösbar verbunden ist, während der Absaugschlauch im Bereich des zweiten Abschnitts keinen Klebstoffauftrag aufweist. Hierdurch kann der Schwammkörper durch den Anwender im Bereich des zweiten Abschnitts vom Ende her bedarfsgemäß eingekürzt werden, um die Länge des Schwammkörpers an die zu behandelnde Wundstelle anzupassen. Es ist auch möglich, dass der Anwender den über dem gesamten zweiten Abschnitt des Absaugschlauchs vorhandenen Schwamm entfernt. Im Zusammenhang mit der hier vorgeschlagenen Ausführungsform können für den Klebstoffauftrag übliche Klebstoffe verwendet werden, welche für medizinische Anwendungen geeignet sind und welche eine stabile Verbindung zwischen Schwammkörper und Absaugschlauch (auch nach einer Sterilisation der Vorrichtung) gewährleisten. Vorzugsweise ist hierbei der erste Abschnitt des Absaugschlauchs im Fluidsystem stromaufwärts des zweiten Abschnitts vorhanden. Der mit einem Klebstoffauftrag versehene erste Abschnitt des Absaugschlauchs ist also im Vergleich zu dem zweiten Abschnitt des Absaugschlauchs näher an der Wundstelle. Die Längenanpassung des Schwammkörpers erfolgt bei dieser besonders bevorzugten Ausführungsform vom stromabwärts vorhandenen Ende her.

Die Längenanpassung wird bei dieser Weiterentwicklung der Erfindung unmittelbar vor Gebrauch des Saugkörpers zur Unterdrucktherapie durchgeführt und kann beispielsweise mittels einer Schere oder mittels eines Skalpells bewerkstelligt werden. Die Längenanpassung kann durch den Anwender, üblicherweise ein Arzt, schnell und einfach durchgeführt werden. Die Vorbereitungszeit für den Eingriff kann verkürzt werden. Der Schwammkörper bleibt während der Unterdruckbehandlung mit dem ersten Abschnitt des Absaugschlauches fest verbunden. Die gemäß dieser besonders vorteilhaften Ausführungsform vorgeschlagene Lösung überwindet aus dem Stand der Technik bekannte Probleme bei der Längenanpassung des Schwammkörpers: Bei einer Längenanpassung des Schwammkörpers vom stromabwärts vorhandenen Ende her konnten bei den aus dem Stand der Technik bekannten Schwammkörpern mit dem Absaugschlauch mittels Klebstoffauftrag verbundene Materialanteile (insbesondere Schaumstoff) nicht vollständig entfernt werden. Bei einer Längenanpassung des Schwammkörpers vom stromaufwärts vorhandenen Ende her wurde die Struktur des wundnahen Endbereichs des Schwammkörpers in unerwünschter Weise verändert. So wurden durch das Zuschneiden des Schwammkörpers vom wundnahen Ende her beispielsweise am Ende vorhandene Greifmittel abgeschnitten. Ein gegebenenfalls aus dem Schwammkörper herausragender Anteil des Absaugschlauches wurde bei den aus dem Stand der Technik bekannten Schwammkörpern abgeschnitten. Ein gegebenenfalls im Schwammkörperende endender Absaugschlauch wurde freigelegt.

Vorzugsweise umfasst der erste Abschnitt des Absaugschlauches bei dieser Ausführungsform 10 % bis 90 % der entlang der Achse des Absaugschlauches gemessenen Summe der Längen von erstem und zweitem Abschnitt des Absaugschlauchs. Besonders bevorzugt umfasst der erste Abschnitt des Absaugschlauches 25 % bis 80 % der entlang der Achse des Absaugschlauches gemessenen Summe der Längen von erstem und zweitem Abschnitt des Absaugschlauchs, insbesondere 40 % bis 70 %. In der Praxis hat es sich als besonders vorteilhaft erwiesen, wenn der erste und der zweite Abschnitt des Absaugschlauches entlang der Achse des Absaugschlauches eine im Wesentlichen gleiche Länge aufweisen. Hierdurch ergibt sich eine vorteilhafte Kombination aus Stabilität der Klebestelle und einer ausreichenden Möglichkeit zur Längenanpassung des Schwammkörpers.

Bei der hier dargestellten Weiterentwicklung der Erfindung kann der Anwender also zunächst aus dem Kit einen Saugkörper auswählen, dessen Schwammkörper einen für die Wundhöhle geeigneten Durchmesser d aufweist. Anschließend kann er, falls erforderlich die gemäß der Ausführungsform vereinfachte Längenanpassung vornehmen.

Der Absaugschlauch kann optional einen weiteren (vierten) Abschnitt umfassen, welcher stromaufwärts des ersten Abschnitts vorhanden ist und welcher von dem Schwammkörper nicht umschlossen ist. Dies bedeutet, dass bei dieser Variante der Erfindung ein Teilstück des Absaugschlauchs wundseitig aus dem Schwammkörper herausragt.

Der an dem Absaugschlauch unlösbar angebrachte Schwammkörper umfasst vorzugsweise einen offenzelligen Polymerschaum, insbesondere einen retikulierten Polymerschaumstoff. Bei dem Polymer handelt es sich vorzugsweise um Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan, Kollagen oder um eine Mischung aus den genannten Polymeren.

In einer besonders bevorzugten Ausführungsform umfasst der Schwammkörper einen offenzelligen Polyurethanschaumstoff, der erhältlich ist durch Umsetzung einer Mischung umfassend die Komponenten (i) Polyisocyanat, (ii) Polyol, bevorzugt Polyesterpolyol, (iii) Treibmittel und (iv) Katalysator.

Als Schaumstoffe werden üblicherweise Werkstoffe mit über die gesamte Masse verteilten Zellen (offen, geschlossen oder beides) verstanden. Somit weisen solche Werkstoffe üblicherweise eine Rohdichte (gemäß DIN EN ISO 845) auf, die niedriger ist als die Dichte der Gerüstsubstanz. Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Schaumstoffs wird üblicherweise nach ASTM D 2856-87, Verfahren B) bestimmt. Unter Zellwand wird üblicherweise die die Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke, des übrigen Bereichs der Zellwand auf. Bei dem offenzelligen Schaumstoff kann es sich um einen retikulierten oder um einen nichtretikulierten Schaumstoff handeln. Unter einem retikulierten Schaumstoff wird ein Schaumstoff verstanden, der im Wesentlichen nur Zellstege aufweist. Bei einem retikulierten Schaumstoff sind die Zellwände somit im Wesentlichen entfernt. Die Retikulierung wird üblicherweise in einer Druckkammer, z.B. einer Stahlkammer durchgeführt. Nach Einbringen des Schaumstoffs in die Stahlkammer wird die Luft abgesaugt (bevorzugt zu 50 bis 100 Gew.-%, mehr bevorzugt zu 70 bis 99 Gew.-%) und durch ein Brenngasgemisch, bevorzugt durch ein Gemisch enthaltend Wasserstoff und Sauerstoff, insbesondere im molaren Verhältnis von 2 : 1 ersetzt. Bei der Zündung des Gasgemisches zerreißen die Zellhäute durch die entstehende Hitze- und Druckwelle. Gegebenenfalls erfolgt auch ein zumindest teilweises Aufschmelzen der Zellstege, so dass diese verstärkt werden.

Es hat sich ferner gezeigt, dass vorstehend genannte Aufgaben unerwartet vorteilhaft gelöst werden können, wenn der im Schwammkörper vorhandene Schaumstoff eine spezielle Luftdurchlässigkeit aufweist. In einer bevorzugten Ausführungsform weist der Schaumstoff eine Luftdurchlässigkeit von 1000 bis 8000 l/(m²sec), mehr bevorzugt von 1500 bis 6000 l/(m²sec), noch mehr bevorzugt von 2000 bis 5000 l/(m²sec), besonders bevorzugt von 2300 bis 4000 l/(m²sec) und insbesondere von 2400 bis 3300 l/(m²sec), gemessen gemäß DIN EN ISO 9237 (20 mm Prüfdicke, 20 cm² Prüffläche, 200 Pa Differenzdruck) auf.

Hierbei weist der im Schwammkörper vorhandene Schaumstoff insbesondere eine Bruchdehnung von 100 % bis 400 %, mehr bevorzugt von 120 % bis 300 %, noch mehr bevorzugt von 150 % bis 200 %, gemessen gemäß DIN 53571, auf.

Gemäß einer weiteren bevorzugten Ausführungsform der Erfindung weist der im Schwammkörper vorhandene Schaumstoff eine Zellzahl (= Anzahl der Poren entlang einer Geraden pro laufendem Inch) von mindestens 5 und höchstens 400 Zellen pro Inch auf. Gemäß einer alternativen, gleichfalls sehr vorteilhaften Ausführungsform, beträgt die Zellzahl des im Schwammkörper vorhandenen Schaumstoffs mindestens 10 und höchstens 200 Zellen pro Inch. Die Zellzahl wird bevorzugt mikroskopisch bestimmt.

Der Schwammkörper kann auch zwei oder mehr konzentrisch vorliegende Lagen, insbesondere zwei oder mehr konzentrisch vorliegende Lagen aus Schaumstoff, umfassen. Hierbei ist es besonders vorteilhaft, wenn die äußerste Lage eine Zellzahl zwischen 5 und 200 Zellen pro Inch, vorzugsweise eine Zellzahl zwischen 6 und 100 Zellen pro Inch, besonders bevorzugt eine Zellzahl zwischen 8 und 30 Zellen pro Inch aufweist. Gleichzeitig sollte eine weiter innen vorhandene zweite Lage vorzugsweise zwischen 10 und 400 Zellen pro Inch, besonders bevorzugt zwischen 15 und 200 Zellen pro Inch und insbesondere zwischen 40 und 60 Zellen pro Inch aufweisen. Bei einem derartigen Schwammkörper, welcher zwei oder mehr konzentrisch vorliegende Lagen aus Schaumstoffen mit einer unterschiedlichen Zellzahl umfasst, kann eine besonders effektive Ableitung von Fluiden im Inneren des Schwammkörpers mit einer besonders vorteilhaft ausgestalteten Wundkontaktfläche kombiniert werden.

Gemäß einer weiteren vorteilhaften Ausführungsform ist vorgesehen, dass die Rohdichte des Schaumstoffs zwischen 10 und 350 kg/m³ beträgt, gemessen gemäß DIN EN ISO 845. Besonders bevorzugt beträgt die Rohdichte 15 bis 65 kg/m³, insbesondere 20 bis 45 kg/m³. Die Stauchhärte des für den Schwammkörper vorgesehenen Schaumstoffs beträgt vorzugsweise mindestens 1,5 kPa und höchstens 22,5 kPa, gemessen nach ISO 3386-1.

Der von dem Kit umfasste Schwammkörper kann mit einem Zusatz und/oder Hilfsstoff beschichtet oder imprägniert sein. Bei dem Zusatz oder der Imprägnierung kann es sich beispielsweise um eine die Wundheilung fördernde Salbengrundlage handeln, wobei die Salbengrundlage vorzugsweise zusätzlich einen Quellstoff umfasst. Exemplarisch wird auf die WO2012/167943 verwiesen, welche im Rahmen der Erfindung geeignete Salbengrundlagen beschreibt. Eine im Rahmen der Erfindung besonders geeignete Salbengrundlage, welche zusätzlich einen Quellstoff umfasst, ist in Beispiel 1 der Patentanmeldung WO2012/167943 beschrieben.

Der von dem Kit umfasste Schwammkörper kann alternativ oder zusätzlich zu der vorstehend beschriebenen Salbengrundlage eine antimikrobiell wirksame Substanz umfassen, beispielsweise Polyhexanid. Bei der antimikrobiell wirksamen Substanz kann es sich auch um ein Antibiotikum handeln, beispielsweise um Oxytetracyclin oder um eine Kombination von Bacitracin und Neomycin. Gegebenenfalls kann der Zusatz oder die Imprägnierung zusätzlich zum Antibiotikum oder alternativ eine entzündungshemmende Substanz umfassen, beispielsweise Hydrocortison.

Gemäß einer besonders bevorzugten Ausführungsform umfasst die antimikrobiell wirksame Substanz Silber oder eine Silber-Verbindung.

Der Schwammkörper kann Silber in Form von Silberionen oder in Form von atomarem Silber enthalten. Bevorzugt ist Silber in Form einer Silberbeschichtung auf der Oberfläche des Schwammkörpers aufgebracht. Alternativ kann das Silber innerhalb des Schwammkörpers verteilt sein. Beispielsweise kann bei offenzelligen Schaumstoffen Silber bereits in die härtbare Zusammensetzung mit eingebracht werden. Bevorzugt enthält der im Schwammkörper vorhandene Schaumstoff 0,000001 bis 0,1 Gew.-% Silber, mehr bevorzugt 0,0001 bis 0,01 Gew.-% Silber, bezogen auf das Gesamtgewicht des jeweiligen Schaumstoffs.

Des Weiteren kann der Schwammkörper einen Partikel-förmigen Zusatz umfassen, wobei es sich bei dem Partikel-förmigen Zusatz insbesondere um ein quellfähiges Polymer handelt. Gemäß einer bevorzugten Ausführungsform umfasst der Schwammkörper Partikel aus einem superabsorbierenden Polymer (SAP).

Der Absaugschlauch umfasst bevorzugt einen für medizinische Zwecke geeigneten Kunststoff, insbesondere Silikon, Polyacetat, Polycarbonat, Polyethylen, Polyurethan, Weich-Polyvinychlorid (PVC, welches durch einen Weichmacher modifiziert wurde, beispielsweise durch Tri-2-ethylhexyl Trimellitate - "TOTM"), Polyvinylalkohol, thermoplastische Polymere oder eine Mischung daraus.

Zur Bereitstellung des während der Therapie erforderlichen Unterdrucks wird der Saugkörper mit einer Unterdruckquelle verbunden. Bei der Unterdruckquelle handelt es sich insbesondere eine Saugflasche oder eine elektrisch antreibbare Unterdruckquelle. Eine im Zusammenhang mit der vorliegenden Erfindung besonders geeignete elektrisch antreibbare Unterdruckquelle ist kommerziell unter der Bezeichnung VivanoTec® erhältlich (Paul Hartmann AG, Heidenheim).

Das Kit kann bei der Unterdrucktherapie von endoluminalen Wundstellen im Bereich des Gastrointestinaltraktes verwendet werden.

Die Verwendung des Kits umfasst die folgenden Schritte, welche die Vorbereitung der Saugkörper (Anpassung der Länge des Schwammkörpers) und die sich daran anschließende Unterdrucktherapie betreffen:
i) Bereitstellen eines Kits, wie in dem vorliegenden Dokument beschrieben, wobei des Weiteren eine Unterdruckquelle bereitgestellt wird.
ii) Endoskopische Vermessung der zu behandelnden Wundstelle bzw. Kavität im Bereich des Gastrointestinaltraktes ("Spiegeln").
iii) Auswahl eines Saugkörpers aus dem Kit, wobei der Saugkörper einen Durchmesser d aufweist, welcher unter Berücksichtigung der ermittelten Abmessung der Wundstelle bzw. Kavität geeignet erscheint.
iv) Verkürzen des Schwammkörpers, falls unter Berücksichtigung der ermittelten Abmessung der Wundstelle bzw. Kavität erforderlich.
v) Einführen des Saugkörpers bis zur Wundstelle bzw. Kavität.
vi) Verbinden des Absaugschlauches mit einer Unterdruckquelle, wobei das Verbinden vorzugsweise mittels eines Konnektors (Schnelltrennverbinder) erfolgt.
vii) Beaufschlagen der Wundstelle bzw. Kavität mit Unterdruck, wobei der Unterdruck über den Absaugschlauch angelegt wird.

Das erfindungsgemäße Kit kann besonders vorteilhaft zur Unterdruckbehandlung im Gastrointestinaltrakt eingesetzt werden. Es eignet sich jedoch gleichermaßen zur Verwendung bei der Unterdrucktherapie von ausgedehnten Wundhöhlen außerhalb des Gastrointestinaltraktes.

### Figuren

- Figur 1 a/b: Medizinisches Kit zur Verwendung bei der endoluminalen Unterdrucktherapie
- Figur 2: Ausführungsform eines in dem Kit enthaltenen Saugkörpers
- Figur 3 a/b: Weitere Ausführungsform eines in dem Kit enthaltenen Saugkörpers
- Figur 4 a/b: Weitere Ausführungsform eines in dem Kit enthaltenen Saugkörpers
- Figur 5: Weitere Ausführungsform eines in dem Kit enthaltenen Saugkörpers
- Figur 6 a/b: An einem endoskopischen Instrument befestigter Saugkörper
- Figur 7a - c: Längenanpassung des Schwammkörpers
- Figur 8 a - e: Rückbildung einer Abszesshöhle während der Unterdruckbehandlung
- Figur 9 a - d: Konnektorelemente

### Bezugszeichen

- 1: Absaugschlauch
- 2: Schwammkörper
- 3: Fassschlaufe
- 4: Perforation
- 5: Materialabschnitt
- 6: Zapfen
- 7: Abschnitt des Schwammkörpers, welcher die Fassschlaufe enthält
- 8: Übergangsbereich zwischen Abschnitt 7 und dem Schwammkörper
- 11: Speiseröhre mit seitlich abzweigender Kavität
- 12: Kavität
- 13: Anastomose
- 14: Speiseröhre
- 15: Erster Abschnitt des Absaugschlauchs
- 16: Zweiter Abschnitt des Absaugschlauchs
- 17: Dritter Abschnitt des Absaugschlauchs
- 18: Vierter Abschnitt des Absaugschlauchs
- 19: Klebstoffauftrag zur Befestigung des Schaumkörpers
- 10, 20, 30, 40, 50, 60, 70: Saugkörper
- 51: Klebstoffauftrag zur Befestigung des Fadens
- 61: Endoskop
- 62: Arbeitskanal
- 63: Mikromechanischer Greifer
- 64: Zange
- 66: Tube mit Gleitmittel
- 90: Konnektor mit einem männlichen und einem weiblichen Konnektorelement
- 91: Männliches Konnektorelement
- 92: Weibliches Konnektorelement
- 93: Schlauchtülle am männlichen Konnektorelement
- 94: Schlauchtülle am weiblichen Konnektorelement
- 95: Vorsprung
- 96: Dichtung (O-Ring)
- 97: Hilfsschlauch
- 98: Übergangsbereich zwischen Absaugschlauch und männlichem Konnektorelement
- 99: Übergangsbereich zwischen Hilfsschlauch und weiblichen Konnektorelement
- 100: Medizinisches Kit
- 101: Übergangsbereich zwischen männlichem Konnektorelement und weiblichen Konnektorelement

### Figuren 1 a/b

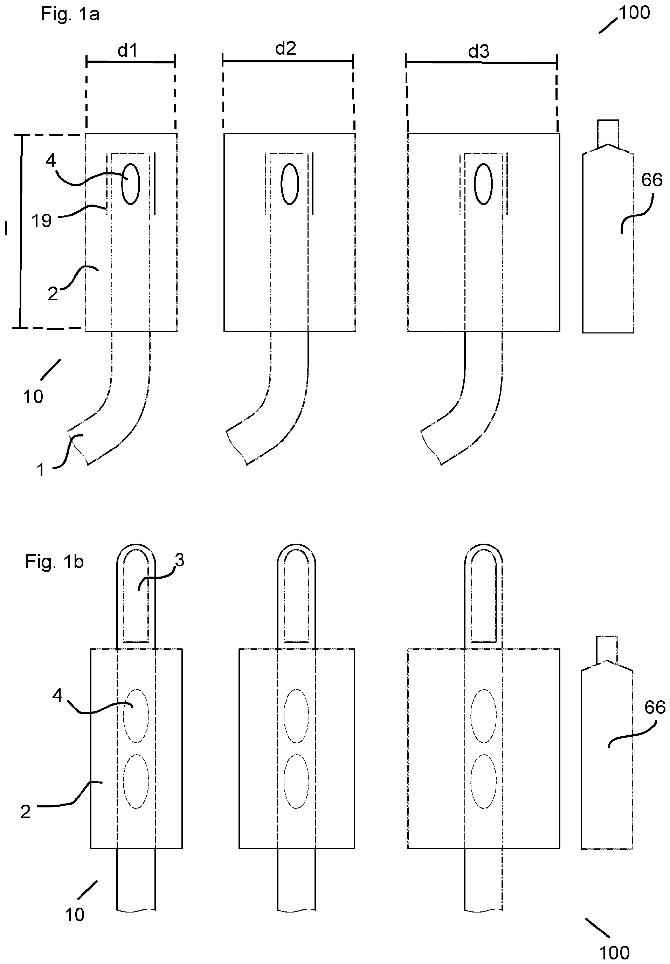

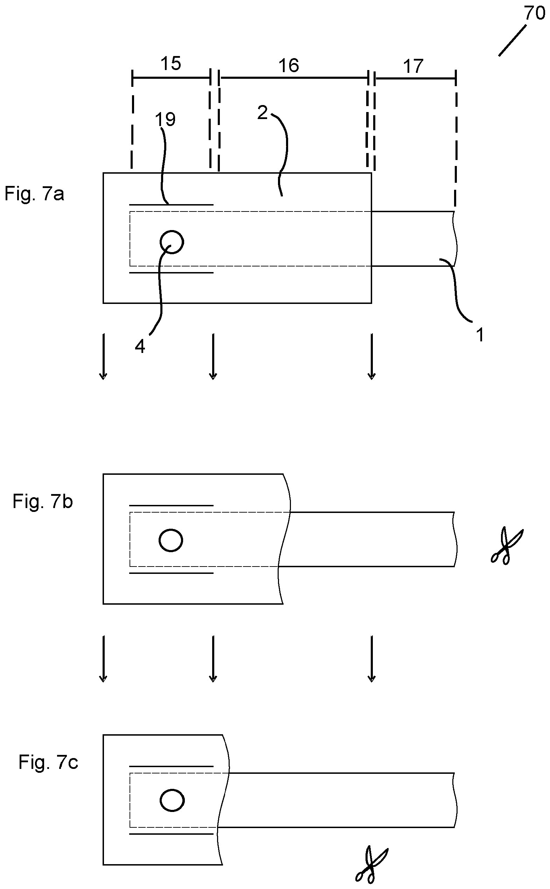

Die Figuren 1 a/b zeigen schematisch ein medizinisches Kit (100), welches zur Verwendung bei der Unterdrucktherapie des Gastrointestinaltraktes vorgesehen ist. Bei den in den Figuren 1 a/b dargestellten Beispielen enthält das Kit jeweils drei Saugkörper (10). Weiterhin enthält das Kit jeweils eine Tube (66) mit Gleitgel. Das Gleitgel kann vor dem Einbringen des Saugkörpers (10) in den Gastrointestinaltrakt auf den Schwammkörper (2) aufgetragen werden, um die Applikation des Saugkörpers (10) zu erleichtern. Vorzugsweise wird das Gleitgel erst aufgebracht, nachdem eine Längenanpassung des Saugkörpers (10) durch den Anwender erfolgt ist, falls erforderlich. Die in den Figuren 1 a/b gezeigten Saugkörper bestehen aus einem Schwammkörper (2) und einem Absaugschlauch (1), wobei der Schwammkörper vorzugsweise einen polymeren Schaumstoff, insbesondere aus Polyester-Polyurethan, umfasst. Der Absaugschlauch (1) weist mindestens eine Öffnung (4) zur Beaufschlagung von Unterdruck und zum Absaugen von Sekreten aus dem Wundbereich auf. Der Schwammkörper (2) ist mittels Klebstoffauftrag (19) an dem Absaugschlauch (1) unlösbar befestigt (Der Klebstoffauftrag (19) ist nur in Figur 1 a und in Figur 7 gezeigt. Gleichwohl ist der Klebstoffauftrag (19) bei allen anderen der in den Figuren 1 - 6 dargestellten Saugkörpern vorhanden, um eine sichere und unlösbare Verbindung von Schwammkörper und Absaugschlauch zu gewährleisten). Hierbei ist besonders vorteilhaft, wenn der Klebstoffauftrag (19), wie in Figur 1a schematisch angedeutet, nur an einem wundnahen Endbereich des vom Schwammkörper (2) umschlossenen Bereichs des Absaugschlauches vorhanden ist. Es ist dann nämlich möglich, Anteile des stromabwärts der Klebestelle vorhanden Schaums durch radial geführte Schnitte wegzuschneiden und so eine Anpassung der Länge des Schwammkörpers an die Wundstelle zu erreichen. Dies wird im Zusammenhang mit Figur 7 noch näher dargestellt. Die Längenanpassung des Schwammkörpers erfolgt unmittelbar vor Gebrauch des Saugkörpers und kann beispielsweise mittels einer Schere oder mittels eines Skalpells durchgeführt werden.

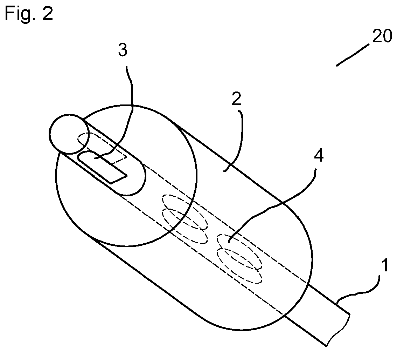

Figur 1 b zeigt eine Ausführungsform der in dem Kit enthaltenen Saugkörper entsprechend dem in Figur 2 gezeigten Beispiel (die Saugkörper weisen eine Fassschlaufe (3) auf).

Bei den in Figuren 1 a/b beispielhaft dargestellten medizinischen Kits weisen alle drei der in dem Kit enthaltenen Schwammkörper jeweils eine identische Länge I auf, während sich die Durchmesser (d₁, d₂, d₃) voneinander unterscheiden. Mit einer derartigen Kombination von Saugkörpern können alle typischen, bei der Unterdrucktherapie des Gastrointestinaltraktes auftretenden Wundsituationen, insbesondere Kavitäten, behandelt werden. Hierzu wählt der Anwender nach der endoskopischen Vermessung des Wundbereichs einen Saugkörper mit einen für die Wundsituation geeigneten Durchmesser d aus. Danach kann der Anwender auf einfache und unkomplizierte Weise den am ausgewählten Saugkörper vorhandenen Schwammkörper in seiner Länge I an die Wundkavität anpassen, indem er den Schwammkörper an seinem stromabwärts vorhandenen Ende zuschneidet.

Die Länge I der Schwammkörper kann bei den in den Figuren 1 a/b gezeigten Kits beispielsweise jeweils 10 cm betragen (herstellerseitige Länge I, also vor einem ggf. erforderlichen Zuschneiden durch den Anwender). Geeignete Abmessungen für den Durchmesser sind beispielsweise: d1 = 1 cm, d2 = 2 cm und d3 = 4 cm.

Vorzugsweise werden herstellerseitig Kits zur Verfügung gestellt, welche entweder zur Verwendung zur Behandlung des oberen oder alternativ zur Behandlung des unteren Gastrointestinaltraktes vorgesehen sind.

### Figur 2

Figur 2 zeigt die perspektivische Darstellung einer Ausgestaltung eines Saugkörpers (20), welcher in dem Kit enthalten sein kann. Der in Figur 2 dargestellte Saugkörper entspricht einem Saugkörper aus dem in Figur 1 b gezeigten Kit. Bei dem Saugkörpers (20) ist zusätzlich eine Fassschlaufe (3) vorhanden, welche das Ergreifen des Saugkörpers (20) durch ein endoskopisches Instrument erleichtert. Die Fassschlaufe wird herstellerseitig durch einen Stanzvorgang direkt aus dem Absaugschlauch (1) hergestellt. In demselben Stanzvorgang, in dem die Fassschlaufe (3) geformt wird, können gleichzeitig weitere Öffnungen (4) in den Absaugschlauch (1) eingebracht werden. Die Öffnungen (4) werden an dem von dem Schwammkörper (2) umschlossen Abschnitt des Absaugschlauches (1) eingebracht und sind zur Applikation des Unterdrucks und zum Ansaugen von Fluiden aus dem Wundraum erforderlich

Die Fassschlaufe (3) ragt bei dem dargestellten Beispiel aus dem Schwammkörper (2) heraus. Die Fassschlaufe ist entsprechend an einem Abschnitt des Absaugschlauchs vorhanden, welcher nicht vom Schwammkörper umschlossen wird. Da die Fassschlaufe (3) bei dem in Figur 2 gezeigten Beispiel mit dem Lumen des Absaugschlauches (1) in einem Kontinuum steht, entsteht während der Behandlung auch ein Sog über die Fassschlaufe (3). Dieser Effekt hat sich als vorteilhaft erwiesen, da die Ableitung zäher oder partikelhaltiger Sekrete hierdurch verbessert wird. Grundsätzlich wäre es jedoch auch möglich, das Lumen des Absaugschlauches (1) gegenüber der Fassschlaufe (3) zu isolieren, so dass der Unterdruck ausschließlich über den Schwammkörper mit dem Wundraum kommunizieren kann (in Figur 2 nicht dargestellt).

Bei dem in der Figur 2 gezeigten Beispiel wurde die Fassschlaufe (3) in einen Schlauch gestanzt, dessen Ende einen abgerundeten und geschlossenen Endabschnitt aufweist. Obgleich es grundsätzlich möglich wäre, die Fassschlaufe (3) in einen Schlauch einzubringen, dessen Endabschnitt offen vorliegt und eine glatte Schnittfläche aufweist (in Figur 2 nicht dargestellt), hat sich ein abgerundeter und geschlossener Abschluss des Absaugschlauches als vorteilhaft erwiesen, da hierdurch eine besonders schonende Einbringung des Saugkörpers (20) in den Wundraum erleichtert wird.

Lediglich beispielhaft wird im Folgenden eine mögliche Dimensionierung für einen Schwammkörper (2), einen Absaugschlauch (1) und die aus dem Absaugschlauch (1) geformte Fassschlaufe (3) angegeben:

| | |
|---|---|
| Außendurchmesser des Absaugschlauchs: | 4,6 mm |
| Wandstärke des Absaugschlauchs: | 1,0 mm |
| Durchmesser des Lumens des Absaugschlauches: | 2,6 mm |
| Länge der Fassschlaufe: | 12 mm |
| Seitliche Ausdehnung der Fassschlaufe: | 2,0 mm |

Der in Figur 2 beispielhaft dargestellte Absaugschlauch (1) ist aus Weich-PVC.

Der Schwammkörper (2) kann beispielsweise einen hydrophoben, retikulierten Polyester-Polyurethan-Schaumstoff umfassen, mit einer Rohdichte gemäß ISO 845 von 26,0 kg/m³, mit einer Stauchhärte gemäß DIN EN ISO 3386-1 von 3,4 kPa, mit einer Bruchdehnung gemäß DIN 53571 von 290 % und mit einer Zellzahl (mikroskopisch bestimmt an einer an der Oberfläche des Schaumes angelegten geraden Linie) von 26 Zellen pro Inch.

Alternativ kann der Schwammkörper (2) einen hydrophoben, retikulierten Polyester-Polyurethan-Schaumstoff umfassen, dessen Rohdichte gemäß ISO 845 28,0 kg/m³, dessen Stauchhärte gemäß DIN EN ISO 3386-1 3,6 kPa, dessen Bruchdehnung gemäß DIN 53571 335 %, und dessen Zellzahl (mikroskopisch bestimmt an einer an der Oberfläche des Schaumes angelegten geraden Linie) 38 Zellen pro Inch beträgt.

Gemäß einer weiteren Ausführungsform kann der Schwammkörper (2) einen hydrophoben Polyether-Polyurethan-Schaumstoff mit einer Rohdichte gemäß ISO 845 von 23,0 kg/m³, mit einer Stauchhärte gemäß DIN EN ISO 3386-1 von 3,8 kPa, mit einer Bruchdehnung gemäß DIN 53571 von 310 %und mit einer Zellzahl (mikroskopisch bestimmt an einer an der Oberfläche des Schaumes angelegten geraden Linie) von 31 Zellen pro Inch umfassen.

Es wäre auch denkbar, dass der Schwammkörper (2) zwei oder mehr konzentrisch angeordnete Lagen aus einem oder mehreren der vorgenannten Schaumstoffe umfasst.

### Figuren 3 a/b

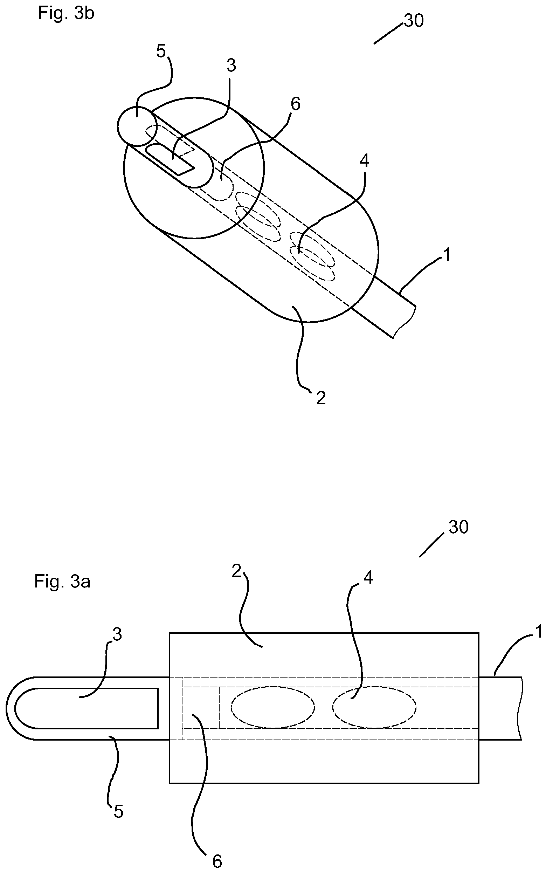

Die Figuren 3 a/b zeigen in schematisierter Darstellung eine weitere Ausführungsform der Erfindung, bei welcher wie bei der in Figur 2 gezeigten Ausführungsform eine Fassschlaufe (3) vorhanden ist. Die Fassschlaufe (3) ist aus einem mit dem wundseitigen Endabschnitt des Absaugschlauchs (1) unlösbar verbundenen Materialabschnitt (5) geformt. Der Materialabschnitt (5) ist an seinem zum Saugkörper (30) hin zeigenden Ende zapfenartig mit dem Absaugschlauch (1) verbunden. Hierzu wird der zapfenförmige Anteil (6) des Materialabschnittes (5) herstellerseitig in den Absaugschlauchs (1) eingeschoben und mit diesem unlösbar verklebt. Der Außendurchmesser des Materialabschnittes (5) sollte vorzugsweise dem Außendurchmesser des Absaugschlauches (1) entsprechen, so dass ein glatter Übergang vom Absaugschlauch (1) zum Materialabschnitt (5) vorhanden ist, wie in Figuren 3 a/b dargestellt. An seinem vom Saugkörper (30) weg zeigenden Ende ist an dem Materialabschnitt (5) eine Fassschlaufe (3) vorhanden. Der Materialabschnitt (5) umfasst vorzugsweise ein Kunststoffmaterial. Im Inneren des Materialabschnittes (5) ist vorzugsweise ein zentrales Lumen (in Figuren 3 a/b nicht dargestellt) vorhanden, welches mit dem Lumen des Absaugschlauches (1) kommunizieren kann. Hierdurch wird der im Absaugschlauch (1) vorhandene Unterdruck bis zur Fassschlaufe (3) weitergeleitet. Dies hat sich als vorteilhaft erwiesen, da hierdurch eine besonders effektive Ableitung von Wundsekreten gewährleistet werden kann. Das vom Absaugschlauch (1) weg zeigende Ende des Materialabschnittes (5) ist vorzugsweise abgerundet ausgeführt, da hierdurch eine schonende Applikation des Saugkörpers (30) in den Wundbereich erleichtert wird.

### Figuren 4 a/b

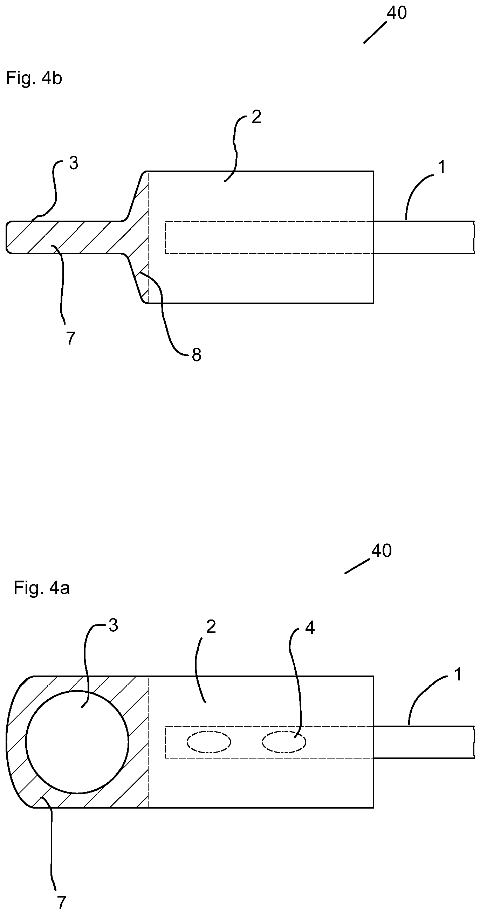

Bei der in den Figuren 4 a/b schematisch gezeigten weiteren Ausführungsform der Erfindung ist gleichfalls eine herstellerseitig bereitgestellte Fassschlaufe (3) vorgesehen. Die Fassschlaufen (3) wird herstellerseitig aus einem Abschnitt des Schwammkörpers (2) geformt. Vorzugsweise umfasst der Schwammkörper (2) einen Polymerkunststoff oder besteht aus einem Polymerkunststoff. Figur 4 a zeigt die Fassschlaufe (3) in der Aufsicht, während sie in Figur 4 b von der Seite gezeigt ist (bei der Darstellung gemäß Figur 4 b wurde der Saugkörper (40) also gegenüber Figur 4 a um 90° entlang der Schlauchachse gedreht). Die Fassschlaufe (3) wurde aus dem zylinderförmigen Schwammkörper (2) durch zwei aufeinander folgende Stanzvorgänge geformt. Bei dem ersten Stanzvorgang werden aus dem Kreiszylinder zwei Abschnitte mit kreissegmentförmigen Querschnitt gestanzt. Vorzugsweise sollte ein abgeschrägter Übergangsbereich (8) zwischen dem planaren Abschnitt (7) und dem zylinderförmiger Schwammkörper (2) vorhanden sein, wie in Figur 4b angedeutet. Vor dem zweiten Stanzvorgang wird der Saugkörper um 90° entlang der Schlauchachse gedreht. Dann wird in den plattenförmig ausgebildeten Bereich (7) eine Öffnung gestanzt, um die Fassschlaufe (3) auszubilden. Die Öffnung kann beispielsweise kreisförmig, wie in Figur 4b dargestellt, ausgebildet sein. Vorzugsweise wird der die Fassschlaufe (3) umfassende Bereich (7) nach dem Stanzen gehärtet, um die Stabilität der Fassschlaufe (3) zu erhöhen. Die Härtung kann beispielsweise durch einen unter Hitzeeinwirkung durchgeführten Pressvorgang oder durch chemische Behandlung des Schaumstoffes erfolgen.

### Figur 5

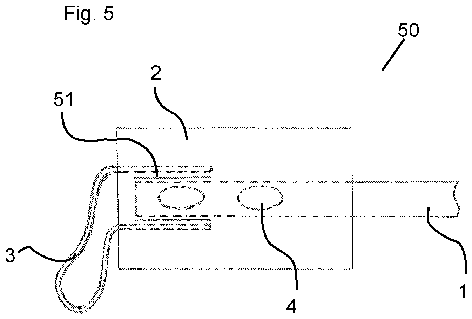

Figur 5 zeigt eine weitere Variante des Saugkörpers, bei welchem die Fassschlaufe (3) aus einem mit dem Absaugschlauch (1) unlösbar verbundenen Materialabschnitt, nämlich aus einem Faden (3), geformt ist. Bei dieser Ausführungsform wird die Fassschlaufe also durch einen Faden (3) gebildet, welcher durch Kleberauftrag (51) unlösbar mit der Außenseite des Absaugschlauches verbunden ist. Der Materialabschnitt (Faden 3) ist mit dem Absaugschlauch nahe seines wundnahen Endes verklebt. Der Faden ragt aus dem Schwammkörper heraus, so dass er von einem endoskopischen Instrument ergriffen werden kann. Zusätzlich zum Kleberauftrag (51), welcher zur unlösbaren Verbindung des Fadens (3) mit dem Absaugschlauch (1) erforderlich ist, ist noch eine weitere Klebeschicht vorhanden, welche den Schwammkörper (2) am Absaugschlauch (1) hält (in Figur 5 nicht gezeigt, siehe jedoch Figuren 1a und 7).

### Figuren 6 a/b

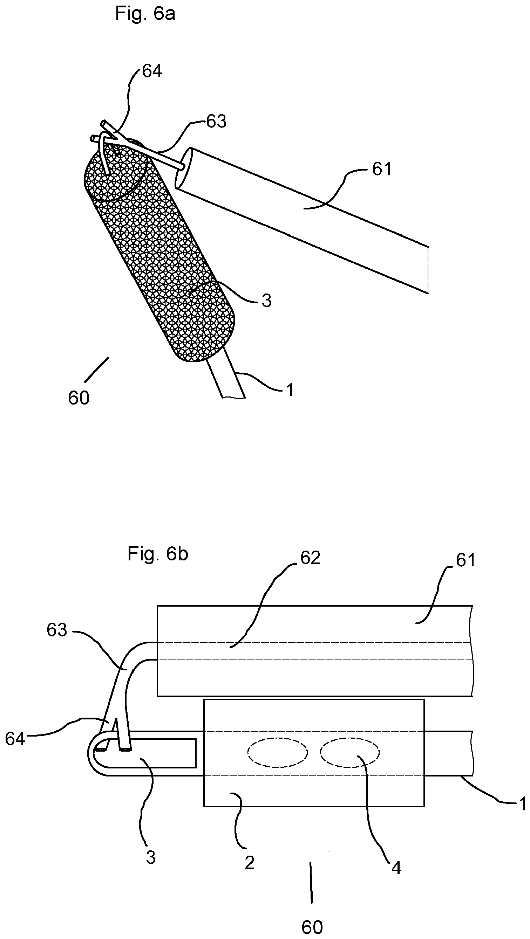

Die schematischen Darstellungen in Figur 6 a/b zeigen einen an einem endoskopischen Instrument (61) lösbar befestigten Saugkörper (60), wobei der Saugkörper eine Fassschlaufe (3) aufweist. Wie aus Figur 6 b erkennbar, verläuft durch das endoskopische Instrument (61) ein Arbeitskanal (62), welcher zum Einführen und Bedienen von mikromechanischen Geräten vorgesehen ist. Bei dem mikromechanischen Gerät handelt es sich im Zusammenhang mit der vorliegenden Erfindung um einen Greifer (63) mit einer abwinkelbaren Spitze (64). Am Ende des Greifers (63) ist eine bewegliche endoskopische Mikro-Zange (64) vorhanden. Die Mikro-Zange (64), welche in den Figuren 6 a/b nur angedeutet ist, kann mechanisch oder elektronisch ferngesteuert werden. Des Weiteren umfasst das endoskopische Instrument (61) eine Kamera (in Figur 6 a/b nicht dargestellt) zur Spiegelung des zu behandelnden Lumen. Der endoskopische Greifer (63) mit endoskopischer Zange (64) kann den Saugkörper (60) an der Fassschlaufe (3) ergreifen. Die Verbindung von endoskopischem Instrument (61) und Saugkörper (60) mittels endoskopischer Zange (64) und Fassschlaufe (3) stellt der behandelnde Arzt noch außerhalb des Patienten her. Die Längsachse des Saugkörpers (60) kann dann parallel zur Längsachse des Endoskops (61) angeordnet werden. Beim Einführen des Endoskops (61) in das Lumen des Gastrointestinaltraktes und gegebenenfalls in einen von dem Lumen abzweigenden Abszess wird der Saugkörper "huckepack" mitgezogen. Am Zielort kann der Arzt die an dem Greifer (63) vorhandene endoskopische Zange (64) öffnen und den Saugkörper freisetzen. Anschließend wird das Endoskop (61) zurückgezogen. Nach der Entfernung des Endoskops (61) kann an den Absaugschlauch (1) Unterdruck angelegt werden.

### Figuren 7 a/b/c

Die Figuren 7 zeigen eine Ausführungsform des erfindungsgegenständlichen Kits. Die hier beispielhaft illustrierte Ausführungsform erleichtert die Längenanpassung der Schwammkörper der in dem Kit enthaltenen Saugkörper, wie bereits im Zusammenhang mit Figur 1 a dargestellt.

Der schematisch wiedergegebene Saugkörper aus Figur 7 a, welcher erfindungsgemäß Bestandteil eines medizinischen Kits ist, umfasst einen Schwammkörper (2) und einen mit dem Schwammkörper mittels Klebstoffauftrag (19) unlösbar verbundenen Absaugschlauch (1). Ein Schwammkörper (2) und ein mit dem Schwammkörper verbundener Absaugschlauch (1) wird im vorliegenden Dokument auch als Saugkörper bezeichnet. Der Schwammkörper (2) wird normalerweise aus einem offenzelligen Polymerkunststoff ("Schaumstoff") hergestellt. Der Absaugschlauch weist mindestens drei Abschnitte (15, 16, 17) auf. Herstellerseitig sind der erste Abschnitt (15) und der zweite Abschnitt (16) des Absaugschlauchs vom Schwammkörper umschlossen, während auf den dritten Abschnitt (17) des Absaugschlauchs kein Schaum aufgebracht wird. Der dritte Abschnitt (17) des Absaugschlauchs soll während der Behandlung aus dem Gastrointestinaltrakt heraus nach außen geführt und mit einer Unterdruckquelle verbunden werden. Der Absaugschlauch (1) weist im Bereich des ersten Abschnitts (15) mindestens eine Öffnung (4) auf. In den stromabwärts angeordneten Abschnitten des Absaugschlauchs (1) können weitere Öffnungen (4) vorhanden sein. Im Bereich des ersten Abschnitts (15) ist der Absaugschlauch (1) mit einem Klebstoffauftrag (19) versehen, welcher eine unlösbare Verbindung zwischen Absaugschlauch Schwammkörper bewirkt. Im Bereich des zweiten Abschnitts(16) des Absaugschlauchs ist herstellerseitig kein Klebstoffauftrag (19) vorgesehen. Entsprechend kann der Schwammkörper, wie in den Figuren 7 a - c dargestellt, von seinem stromabwärts gelegenen Ende her schrittweise verkürzt werden. Diese Längenanpassung wird erst von dem Anwender (normalerweise ein/eine Arzt/Ärztin) kurz vor der Verwendung der Vorrichtung durchgeführt. Die Längenanpassung des Saugkörpers wird anhand einer vor dem Eingriff durchgeführten endoskopischen Vermessung der Wundstelle vorgenommen.

Bei dem in Figur 7b gezeigten Saugkörper wurde ein Ring-Segment des Schaumstoff-Körpers (2) vom Anwender mittels Schere oder Skalpell entfernt. Der zur Wundstelle hin orientierte stromaufwärts vorhandene Anteil des Schwammkörpers wird bei der in den Figuren 7 a -c gezeigten Ausführungsform der Erfindung durch die Längenanpassung nicht modifiziert. Dies setzt voraus, dass wie in Figur 7 a dargestellt, der erste Abschnitt (15) des Absaugschlauchs stromaufwärts des zweiten Abschnitts (16) des Absaugschlauchs angeordnet ist. Eine umgekehrte Anordnung (nicht dargestellt) von erstem und zweitem Abschnitt wäre denkbar, wobei dann eine Längenanpassung nur an dem stromaufwärts vorhandenen zweiten Abschnitt vorgenommen werden kann. Figur 7 c zeigt beispielhaft eine durch den Anwender vorgenommene Längenanpassung, bei der der um den zweiten Abschnitt (16) des Absaugschlauchs ursprünglich vorhandene Schaum (2) weitestgehend vollständig abgeschnitten wurde.

### Figuren 8 a - e

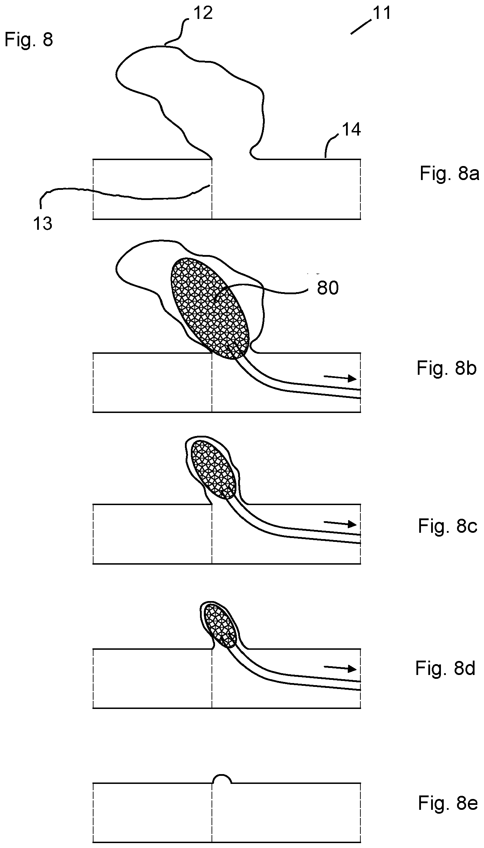

Figuren 8 a - e zeigen einen durch Unterdruckbehandlung unterstützten Heilungsverlauf bei einer aus einer Speiseröhre (14) abzweigenden Kavität (12). Für die in den Figuren 8 a - e gezeigte Unterdruckbehandlung einer endoluminalen Kavität, welche an sich aus dem Stand der Technik bekannt ist, kann besonders vorteilhaft ein hinsichtlich seiner Dimensionierung geeigneter Saugkörper aus dem erfindungsgemäßen Kit ausgewählt werden. Die in den Figuren 8 a - e dargestellten Saugkörper (80) sind lediglich schematisch gezeichnet. Eine aus der Speiseröhre seitlich abzweigende Kavität (12) kann unter anderem infolge einer chirurgisch erzeugten Anastomose (13) entstehen (beispielsweise nach einer Tumorexzision). Vor der Unterdruckbehandlung wird die Kavität (12) endoskopisch gespiegelt (nicht dargestellt), um ihre Abmessung zu ermitteln. Anhand der endoskopischen Vermessung erfolgt eine Längenanpassung des Schwammkörpers, wobei die Längenanpassung besonders einfach unter Einsatz der in Figur 7 gezeigten Saugkörper vorgenommen werden kann. Nachdem ein derart an die Abmessungen der Kavität angepasster Saugkörper (80) in die Wundhöhle (12) eingebracht ist, kann über den Absaugschlauch (1) Unterdruck angelegt werden (Figur 8 b). Die Unterdruckapplikation bewirkt eine Ableitung von Wundsekreten und unterstützt die Kontraktion der Kavität (12), wie in Figuren 8 b bis 8 d angedeutet. Nach einer permanenten Unterdruckbehandlung von einigen Tagen bis zu mehreren Wochen ist die Kavität bereits so weit geschrumpft, dass der Fluidsammelkörper aus den Wundbereich entfernt werden kann (Figur 8 e).

### Figuren 9 a - d

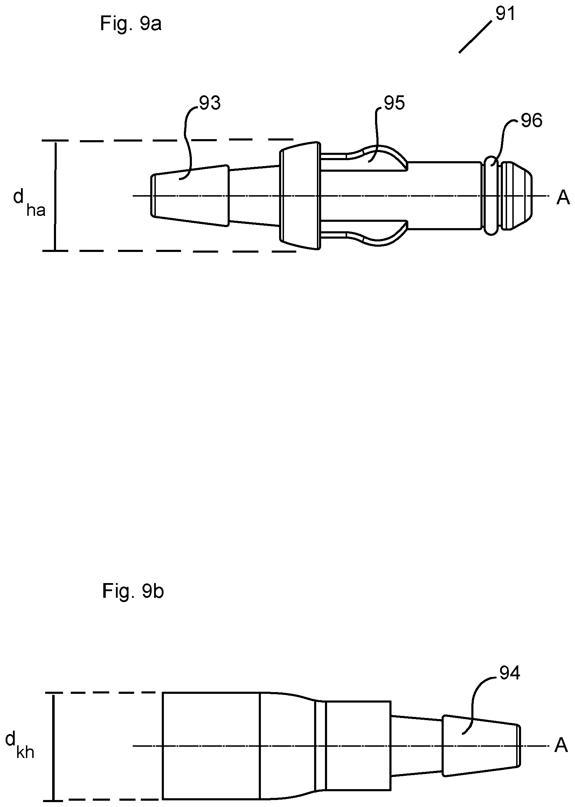

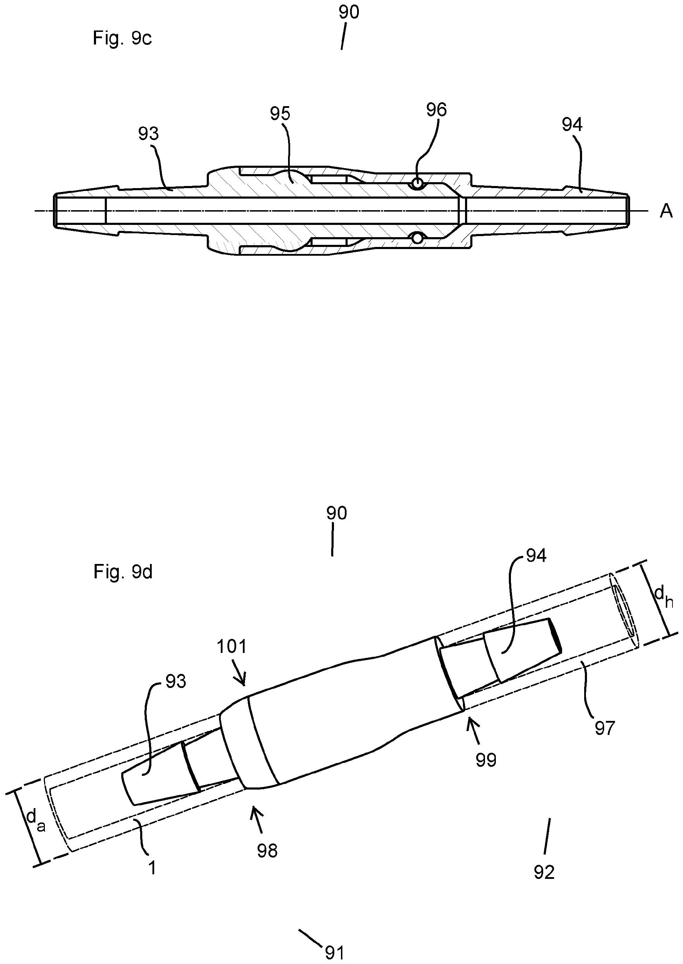

Die Figuren 9 a - d zeigen Konnektorelemente, welche besonders vorteilhaft zur Translokation des aus dem Mund des Patienten herausragenden Absaugschlauches eingesetzt werden können.

In den Figur 9 a - d ist der Absaugschlauch beispielhaft an dem männlichen Konnektorelement befestigt, während der Hilfsschlauch mit dem weiblichen Konnektorelement verbunden ist. Dies kann jedoch auch umgekehrt erfolgen.

Figur 9 a zeigt in der Aufsicht eine mögliche Ausgestaltung eines männlichen Konnektorelementes, welches eine Schlauchtülle (93) zum Aufstecken des Absaugschlauches und ein männliches Steckerelement zum Koppeln mit dem weiblichen Konnektorelement (92) umfasst. Am Steckerelement ist ein Dichtungsring ("O-Ring") und ein flexibler Vorsprung (95) vorhanden. Der Vorsprung (95) kann in eine am weiblichen Konnektorelement vorhandene Mulde eingreifen, wie in Figur 9 c erkennbar.

Figur 9 b zeigt das weibliche Konnektorelement, welches gleichfalls eine Schlauchtülle (94) aufweist, in der Aufsicht. Der maximale Durchmesser der Konnektorelemente (91, 92), also der Bereich maximaler Ausdehnung quer zu der in den Figuren 9 a - c durch eine gestrichelte Linie angedeuteten Längsachse A, wird vorliegend als dₖₐ (Durchmesser des am Absaugschlauch vorhandenen Konnektorelementes) bzw. dₖₕ (Durchmesser des am Hilfsschlauch vorhandenen Konnektorelementes) bezeichnet.

Die miteinander verbundenen Konnektorelemente (91, 92) sind in Figur 9 c im Längsschnitt und in Figur 9 d in der Aufsicht gezeigt. In Figur 9d sind der auf das Konnektorelement (91) aufgesteckte Absaugschlauch (1) und der an das Konnektorelement (92) aufgesteckte Hilfsschlauch (97) angedeutet. Der Durchmesser des Absaugschlauches wird als dₐ und der Durchmesser des Hilfsschlauches als dₕ bezeichnet. Wesentlich für die vorgeschlagenen Konnektorelemente (91, 92) ist einerseits, dass sie hinsichtlich ihrer Dicke, also hinsichtlich ihrer maximalen Durchmesser (dₖₐ, dₖₕ) die Durchmesser (dₐ, dₕ) der mit den Konnektorelementen (91, 92) verbundenen Schläuche (1, 97) nicht wesentlich, insbesondere nicht um mehr als den Faktor 1,5 , bevorzugt um nicht um mehr als den Faktor 1,4 , besonders bevorzugt um nicht um mehr als den Faktor 1,2 überschreiten. Anderseits muss der Übergang (98, 99) zwischen jedem der Schläuche und seinem korrespondierendem Konnektorelement fließend ausgebildet sein. Es dürfen insbesondere keine Stufen oder Kanten vorhanden sein. Des Weiteren muss auch der Übergangsbereich (101) zwischen den beiden Konnektorelementen (91, 92) fließend ausgestaltet sein. Die vorgenannten Eigenschaften (Durchmesser, fließende Übergänge) gewährleisten, dass die aneinander gekoppelten Konnektorelemente (91, 92) sanft und ohne Verletzungsgefahr bei der eingangs erklärten Translokation durch den Nasengang gezogen werden können.

## Patentansprüche

1. Medizinisches Kit (100) zur Verwendung bei der Unterdrucktherapie von endoluminalen Wundstellen im Bereich des Gastrointestinaltraktes,
umfassend mindestens zwei Saugkörper (10, 20, 30, 40, 50, 60, 70),
wobei jeder der mindestens zwei Saugkörper (10, 20, 30, 40, 50, 60, 70) einen Schwammkörper (2) und einen mit dem Schwammkörper (2) mittels Klebstoffauftrag (19) unlösbar verbundenen Absaugschlauch (1) umfasst,
wobei jeder der Schwammkörper (2) im Wesentlichen zylinderförmig ausgebildet ist, insbesondere als senkrechter Kreiszylinder,
wobei jeder der Schwammkörper (2) einen Durchmesser d und eine Länge I aufweist,
wobei in dem Absaugschlauch (1) mindestens eine Öffnung (4) vorhanden ist,
und wobei sich jeder der in dem Kit (100) vorhandenen Schwammkörper (2) in mindestens einer der Größen ausgewählt aus Durchmesser d und/oder Länge I von jedem anderen der in dem Kit (100) vorhandenen Schwammkörper (2) unterscheidet,
**dadurch gekennzeichnet,**
**dass** sich mindestens zwei der in dem Kit (100) vorhandenen Schwammkörper (2) in nur einer einzigen Größe ausgewählt aus Durchmesser d oder Länge I von jedem der anderen in dem Kit (100) vorhandenen Schwammkörper (2) unterscheiden,
wobei das Kit (100) mindestens zwei Schwammkörper (2) umfasst, welche eine identische Länge I und einen unterschiedlichen Durchmesser d aufweisen.

2. Kit (100) nach Anspruch 1, wobei sämtliche Bestandteile des Kits (100) steril abgepackt vorliegen.

3. Kit (100) nach Anspruch 1 oder 2, wobei das Kit (100) weiterhin ein Gleitgel (66) zum Einführen des Schwammkörpers (2) in ein Lumen des Gastrointestinaltraktes umfasst.

4. Kit (100) nach einem oder mehreren der vorangehenden Ansprüche, wobei an jedem der in dem Kit (100) vorhandenen Absaugschläuche (1) ein Konnektor (90) zum Verbinden des Absaugschlauches (1) mit einer Unterdruckquelle angebracht ist.

5. Kit (100) nach einem oder mehreren der vorangehenden Ansprüche, wobei jeder der in dem Kit (100) vorhandenen Schwammkörper (2) einen offenzelligen Schaum, insbesondere einen offenzelligen Polyurethanschaum, umfasst.

6. Kit (100) nach einem oder mehreren der vorangehenden Ansprüche, wobei jeder der in dem Kit (100) vorhandenen Absaugschläuche (1) Silikon, Polyacetat, Polycarbonat, Polyethylen, Polyurethan, Weich-Polyvinychlorid, Polyvinylalkohol, thermoplastische Polymere oder eine Mischung daraus umfasst.

7. Kit (100) nach einem oder mehreren der vorangehenden Ansprüche, wobei das Kit (100) drei oder vier Saugkörper (10, 20, 30, 40, 50, 60, 70) enthält, und wobei die Länge I der an den Saugkörpern (10, 20, 30, 40, 50, 60, 70) vorhandenen Schwammkörper (2) 5 cm bis 15 cm beträgt.

8. Kit (100) nach Anspruch 7, wobei die Länge I der drei oder vier Schwammkörper (2) 7 cm bis 11 cm beträgt.

9. Kit (100) nach Anspruch 7 oder 8, wobei die unterschiedlichen Durchmesser d der drei oder vier Schwammkörper (2) im Bereich von 0,5 cm bis 10 cm, insbesondere im Bereich von 1 cm bis 5 cm, liegen.

10. Kit (100) nach einem oder mehreren der vorangehenden Ansprüche, wobei an sämtlichen in den Kits (100) enthaltenen Schwammkörpern (2) eine bereits herstellerseitig angebrachte Fassschlaufe (3) vorhanden ist,
und wobei die Fassschlaufe (3) aus einem Abschnitt des Schwammkörpers (2) geformt ist, aus einem Abschnitt des Absaugschlauchs (1) geformt ist oder aus einem mit dem Absaugschlauch (1) unlösbar verbundenem Materialabschnitt (5) geformt ist.

11. Set umfassend mindestens zwei Kits (100) nach einem oder mehreren der vorangehenden Ansprüche, wobei sich die Kits (100) bezüglich der Länge I der in den Kits (100) enthaltenen Saugkörper (10, 20, 30, 40, 50, 60, 70) voneinander unterscheiden.

## Claims

1. Medical kit (100) for use in negative pressure therapy of endoluminal wound sites in the gastrointestinal tract,
comprising at least two absorbent bodies (10, 20, 30, 40, 50, 60, 70),
wherein each of the at least two absorbent bodies (10, 20, 30, 40, 50, 60, 70) comprises a sponge body (2) and a suction hose (1) connected non-detachably to the sponge body (2) by means of adhesive (19), wherein each of the sponge bodies (2) is substantially cylindrical, in particular in the form of a vertical circular cylinder,
wherein each of the sponge bodies (2) has a diameter d and a length 1,
wherein at least one opening (4) is present in the suction hose (1),
and wherein each of the sponge bodies (2) present in the kit (100) differs, in at least one of the sizes chosen from diameter d and/or length 1, from every other of the sponge bodies (2) present in the kit (100),
**characterized in that** at least two of the sponge bodies (2) present in the kit (100) differ, in only a single size chosen from diameter d or length 1, from each of the other sponge bodies (2) present in the kit (100),
wherein the kit (100) comprises at least two sponge bodies (2) that have an identical length 1 and a different diameter d.

2. Kit (100) according to Claim 1, wherein all the constituent parts of the kit (100) are present in sterile packaging.

3. Kit (100) according to Claim 1 or 2, wherein the kit (100) further comprises a lubricant gel (66) for inserting the sponge body (2) into a lumen of the gastrointestinal tract.

4. Kit (100) according to one or more of the preceding claims, wherein a connector (90) for connecting the suction hose (1) to a vacuum source is attached to each of the suction hoses (1) provided in the kit (100).

5. Kit (100) according to one or more of the preceding claims, wherein each of the sponge bodies (2) present in the kit (100) comprises an open-cell foam, in particular an open-cell polyurethane foam.

6. Kit (100) according to one or more of the preceding claims, wherein each of the suction hoses (1) present in the kit (100) comprises silicone, polyacetate, polycarbonate, polyethylene, polyurethane, soft polyvinyl chloride, polyvinyl alcohol, thermoplastic polymers or a mixture thereof.

7. Kit (100) according to one or more of the preceding claims, wherein the kit (100) contains three or four absorbent bodies (10, 20, 30, 40, 50, 60, 70), and wherein the length 1 of the sponge bodies (2) present on the absorbent bodies (10, 20, 30, 40, 50, 60, 70) is 5 cm to 15 cm.

8. Kit (100) according to Claim 7, wherein the length l of the three or four sponge bodies (2) is 7 cm to 11 cm.

9. Kit (100) according to Claim 7 or 8, wherein the different diameters d of the three or four sponge bodies (2) are in the range of 0.5 cm to 10 cm, in particular in the range of 1 cm to 5 cm.

10. Kit (100) according to one or more of the preceding claims, wherein a gripping loop (3), already fitted at the production stage, is present on all of the sponge bodies (2) contained in the kits (100),
and wherein the gripping loop (3) is formed from a portion of the sponge body (2), is formed from a portion of the suction hose (1) or is formed from a material portion (5) connected non-detachably to the suction hose (1).

11. Set comprising at least two kits (100) according to one or more of the preceding claims, wherein the kits (100) differ from one another in terms of the length 1 of the absorbent bodies (10, 20, 30, 40, 50, 60, 70) contained in the kits (100).

## Revendications

1. Kit médical (100) destiné à être utilisé lors de la thérapie par pression négative de plaies endoluminales dans la région du tractus gastro-intestinal,
comprenant au moins deux corps absorbants (10, 20, 30, 40, 50, 60, 70),
chacun des au moins deux corps absorbants (10, 20, 30, 40, 50, 60, 70) comprenant un corps en éponge (2) et un tuyau d'aspiration (1) relié de manière non détachable au corps en éponge (2) au moyen d'une couche d'adhésif (19),
chacun des corps en éponge (2) étant configuré sous forme essentiellement cylindrique, notamment sous la forme d'un cylindre circulaire vertical,
chacun des corps en éponge (2) présentant un diamètre d et une longueur l,
au moins une ouverture (4) étant présente dans le tuyau d'aspiration (1),
et
chacun des corps en éponge (2) présents dans le kit (100) différant au niveau d'au moins une des grandeurs choisies parmi le diamètre d et/ou la longueur l de chaque autre des corps en éponge (2) présents dans le kit (100),
**caractérisé en ce que**
au moins deux des corps en éponge (2) présents dans le kit (100) diffèrent au niveau de seulement une grandeur unique choisie parmi le diamètre d ou la longueur l de chacun des autres corps en éponge (2) présents dans le kit (100),
le kit (100) comprenant au moins deux corps en éponge (2) qui présentent une longueur l identique et un diamètre d différent.

2. Kit (100) selon la revendication 1, dans lequel tous les constituants du kit (100) se présentent sous forme emballée stérile.

3. Kit (100) selon la revendication 1 ou 2, dans lequel le kit (100) comprend en outre un gel lubrifiant (66) pour l'insertion du corps en éponge (2) dans un lumen du tractus gastro-intestinal.

4. Kit (100) selon une ou plusieurs des revendications précédentes, dans lequel un connecteur (90) pour le raccordement du tuyau d'aspiration (1) à une source de pression négative est disposé sur chacun des tuyaux d'aspiration (1) présents dans le kit (100).

5. Kit (100) selon une ou plusieurs des revendications précédentes, dans lequel chacun des corps en éponge (2) présents dans le kit (100) comprend une mousse à cellules ouvertes, notamment une mousse de polyuréthane à cellules ouvertes.

6. Kit (100) selon une ou plusieurs des revendications précédentes, dans lequel chacun des tuyaux d'aspiration (1) présents dans le kit (100) comprend de la silicone, du polyacétate, du polycarbonate, du polyéthylène, du polyuréthane, du polychlorure de vinyle souple, de l'alcool polyvinylique, des polymères thermoplastiques ou un mélange de ceux-ci.

7. Kit (100) selon une ou plusieurs des revendications précédentes, dans lequel le kit (100) contient trois ou quatre corps absorbants (10, 20, 30, 40, 50, 60, 70), et dans lequel la longueur l des corps en éponge (2) présents sur les corps absorbants (10, 20, 30, 40, 50, 60, 70) est de 5 cm à 15 cm.

8. Kit (100) selon la revendication 7, dans lequel la longueur l des trois ou quatre corps en éponge (2) est de 7 cm à 11 cm.

9. Kit (100) selon la revendication 7 ou 8, dans lequel les différents diamètres d des trois ou quatre corps en éponge (2) se situent dans la plage allant de 0,5 cm à 10 cm, notamment dans la plage allant de 1 cm à 5 cm.

10. Kit (100) selon une ou plusieurs des revendications précédentes, dans lequel une boucle de préhension (3) déjà mise en place par le fabricant est présente sur tous les corps en éponge (2) contenus dans le kit (100),
et dans lequel la boucle de préhension (3) est formée à partir d'une section du corps en éponge (2), est formée à partir d'une section du tuyau d'aspiration (1) ou est formée à partir d'une section de matériau (5) reliée de manière non détachable avec le tuyau d'aspiration (1).

11. Ensemble comprenant au moins deux kits (100) selon une ou plusieurs des revendications précédentes, dans lequel les kits (100) diffèrent les uns des autres au regard de la longueur l des corps absorbants (10, 20, 30, 40, 50, 60, 70) contenus dans les kits (100).
